# EUROPÄISCHE PATENTSCHRIFT

(11) **EP 3 630 334 B1**
(45) Veröffentlichungstag und Bekanntmachung des Hinweises auf die Patenterteilung: **03.01.2024**
(21) Anmeldenummer: 18725469.3
(22) Anmeldetag: 15.05.2018
(51) Int. Cl.: B01D 35/30, B01D 29/52, B01D 29/56

(54) **VORKONFIGURIERTE EINWEG-FILTRATIONSVORRICHTUNG**
PRECONFIGURED DISPOSABLE FILTRATION DEVICE
DISPOSITIF DE FILTRATION À USAGE UNIQUE PRÉCONFIGURÉ

(30) Priorität: 22.05.2017 DE 102017111133
(43) Veröffentlichungstag der Anmeldung: 08.04.2020
(73) Patentinhaber: Sartorius Stedim Biotech GmbH, 37079 Göttingen (DE)
(72) Erfinder: LOEWE, Thomas, 37077 Goettingen (DE); DELL, André, 37434 Gieboldehausen (DE); HANDT, Sebastian, 37085 Goettingen (DE); SOMMER, Maik, 37136 Seeburg (DE); FRIESE, Thomas, 99752 Bleicherode (DE); DIXIT, Mandar, Medford, New York 11763 (US); DIEL, Bernhard, 37127 Dransfeld (DE)
(74) Vertreter: Vigand, Philippe
(86) Internationale Anmeldenummer: PCT/EP2018/062598
(87) Internationale Veröffentlichungsnummer: WO 2018/215246

(56) Entgegenhaltungen:
- WO-A1-2015/058821
- WO-A1-2016/033553
- WO-A1-2017/032560
- WO-A2-2014/088882
- US-A1- 2005 045 552
- US-A1- 2015 252 934

## Beschreibung

Die Erfindung betrifft eine vorkonfigurierte Einweg-Filtrationsvorrichtung, insbesondere für großvolumige Filtrationsprozesse.

Allgemein finden in der pharmazeutischen Fertigung von hochwertigen Wirkstoffen Einweg-Prozesse aufgrund der damit erreichbaren hohen Flexibilität sowie Einsparung von Zeit, Investitionen und Betriebsaufwand wie Reinigung und deren Validierung und Überprüfung zunehmend Verbreitung. Hierfür verwendete Einweg-Systeme ("disposables", "single use systems") sind abzugrenzen von wiederverwendbaren Systemen, die nach einmaliger Benutzung nicht einfach entsorgt werden können, sondern für jeden weiteren Einsatz erneut gereinigt, sterilisiert und getestet werden müssen. Einweg-Systeme werden nicht nur für kleinvolumige Prozesse, sondern auch für einen größeren Maßstab gewünscht, wobei die Kosten für solche Systeme nicht unrealistisch hoch anwachsen sollen.

Bei Prozessen mit Bioreaktoren, welche inzwischen auch als Einweg-Reaktoren in Größen von 500, 1.000 und 2.000 Litern zur Verfügung stehen, besteht ein Bedarf für Medienfiltration sowie der Zellernte nachgeschaltete (post harvest) Filtration. Ausgehend von einem Filtrationsgesamtvolumen von mehreren tausend Litern besteht ein Bedarf an Filtern mit entsprechend hohen Gesamtfilterflächen. Derart groß angelegte Filtrationsprozesse mit Gesamtfilterflächen von bis zu 50 m² werden derzeit mit Vorrichtungen durchgeführt, bei denen Filterkerzen in Edelstahlgehäusen zum Einsatz kommen (z. B. Multiround-Systeme). Die Vorrichtungen sind also immer noch auf Wiederverwendung ausgelegt. Dadurch entstehen Nachteile wie geringe Flexibilität, hoher Reinigungsaufwand, durch die Reinigung bedingte Produktionsstopps, etc. Entsprechende Einweg-Lösungen standen in dieser Größenordnung bislang nicht zur Verfügung.

Abhilfe hat die in der WO 2017/032560 A1 vorgestellte, vollständig vorsterilisierbare, anschlussfertige und integritätstestbare Einweg-Filtrationsvorrichtung geschaffen, die für großvolumige Filtrationsprozesse ausgelegt ist. Diese Einweg-Filtrationsvorrichtung umfasst eine Mehrzahl von Einweg-Filtercapsulen einer Standardgröße, die durch Leitungen miteinander verbunden sind und von einer starren Halterung getragen werden. Es können auch unterschiedliche Typen von Filtercapsulen innerhalb derselben Vorrichtung zum Einsatz kommen. Die Leitungen zwischen den Filtercapsulen können als starre Rohrleitungen ausgebildet oder durch mehrere einheitliche Zu- und/oder Ablaufeinrichtungen gebildet sein.

Die EP 2 915 571 A1 zeigt eine wiederverwendbare Filtrationsvorrichtung mit einer Rahmenkonstruktion zur Aufnahme mehrerer Filtrationsmodule. Die einzelnen Filtrationsmodule können mit Verteilerrohren zu einer parallelen Anordnung, einer Serienanordnung oder einer Kombination aus beiden verbunden werden.

Aus der DE 33 21 038 C2 ist es bekannt, Gruppen von wiederverwendbaren Patronenfiltern, die jeweils mehrere Filterpatronen enthalten, durch Ventile parallel oder hintereinander zu schalten.

Die US 4 909 937 A zeigt eine wiederverwendbare Filtrationsvorrichtung, bei der einzelne Filterbereiche wahlweise auf Integrität getestet werden können. Hierzu ist ein Zwischenbereich zwischen Filterkassetten vorgesehen, der mit einem Fluid zum Spülen, Sterilisieren etc. befüllt werden kann. Dieser Zwischenbereich ist über ein Verbindungsstück angeschlossen. Sämtliche Verbindungen und Zuführleitungen können mit Absperrventilen versehen sein.

Aus der EP 0 051 373 A2 ist eine wiederverwendbare Vorrichtung zum Testen von Filtern im selben Gehäuse bekannt, in dem auch der bestimmungsgemäße Filtrationsprozess stattfindet.

Die WO 2014/088882 A2 stellt ein redundantes Filtersystem vor, das mindestens einen Barrierefilter als Prozessfilter umfasst, wobei der Barrierefilter sowohl hydrophile als auch hydrophobe Pfade aufweist, die sowohl Flüssigkeitsals auch Gasdurchlässigkeit ermöglichen. Das Filtersystem umfasst ein Netzwerk von Leitungen und Behältern, wobei das Netzwerk an einem Ende flüssiges Ausgangsmaterial aufnimmt, es durch einen vorgegebenen Prozesspfad leitet und an einem anderen Ende das gewünschte flüssige Produkt ausgibt. Das Netzwerk kann mit einem oder mehreren Eingängen zum Einführen von flüssigem Rohmaterial in den Fluid-Prozessstrom und mit einem oder mehreren Ausgangsanschlüssen zum Ablassen von Fluid aus dem Fluid-Prozessstrom versehen sein.

Die US 2015/252934 A1 offenbart eine Filtrationsvorrichtung mit den Merkmalen des Oberbegriffs des Anspruchs 1. Mehrere über Rohrleitungssysteme verbundene Filtermodule sind in einer einstellbaren Rahmenanordnung gehalten. Die Rohrleitungssysteme können abhängig davon variieren, ob die Filtermodule in einer parallelen Anordnung, einer Reihenanordnung oder einer Parallel-ReihenKombination angeordnet sind. Beispielsweise kann die Filtervorrichtung so konfiguriert sein, dass ein erster Satz von Filtermodulen mit einem zweiten Satz von Filtermodulen strömungstechnisch in Reihe gekoppelt ist.

Die WO 2016/033553 A1 zeigt ein Single-Pass-Tangentialflussfiltrationssystem mit mehreren Filtrationsmodulen, die jeweils mehrere durch Verteilersegmente fluidisch miteinander verbundene Filtercapsulen aufweisen. Das System weist einen Strömungspfad auf, der parallel in die Filtercapsulen in jedem Filtrationsmodul und seriell durch die Verteilersegmente in benachbarten Filtrationsmodulen verläuft. Eine oder mehrere Komponenten des Systems können Einweg-Komponenten sein.

Die übergeordnete Aufgabe der Erfindung besteht darin, eine für großvolumige Filtrationsprozesse ausgelegte Einweg-Filtrationsvorrichtung bereitzustellen, die individuell auf einen gewünschten Filtrationsprozess und dessen Anforderungen ausgelegt werden kann.

Gelöst wird diese Aufgabe durch eine Einweg-Filtrationsvorrichtung mit den Merkmalen des Anspruchs 1. Vorteilhafte und zweckmäßige Ausgestaltungen der erfindungsgemäßen Einweg-Filtrationsvorrichtung sind in den Unteransprüchen angegeben.

Die erfindungsgemäße Einweg-Filtrationsvorrichtung umfasst eine Mehrzahl durch starre Leitungen miteinander verbundener Einweg-Filtercapsulen, von denen wenigstens ein Teil in einem durch eine Halterung universell vorgegebenen Raster fest angebracht ist. Die Filtercapsulen, insbesondere hinsichtlich Filterart, Bauart und/oder Baugröße, und/oder die Verbindungen der Filtercapsulen sind für einen gewünschten Filtrationsprozess vorkonfiguriert. Die Leitungen bilden mehrere Leitungszweige mit zugehörigen Filtercapsulen, die nacheinander oder parallel durchströmt werden.

Die Erfindung beruht auf der Erkenntnis, dass bei einer großen Einweg-Filtrationsvorrichtung mit einer geeigneten Halterung, die grundsätzlich verschiedene Einweg-Filtercapsulen aufnehmen kann, und mit geeigneten Leitungsbauteilen, die verschiedene Strömungswege zur variablen Verbindung der Filtercapsulen ermöglichen, effiziente Aufbauten zur Durchführung spezieller, individuell gestalteter Filtrationsprozesse realisierbar sind. Die erfindungsgemäße Einweg-Filtrationsvorrichtung ist insbesondere hinsichtlich Platzbedarf und Handhabung optimiert. Aufgrund des vorgegebenen Rasters für die Filtercapsulen können die vorzugsweise starren Verbindungsleitungen sehr kurz ausfallen, sodass der Material- und Montageaufwand minimiert ist. Die modular aufgebaute Einweg-Filtrationsvorrichtung kann nach der Bestückung mit den Filtercapsulen und deren Verbindungen als Ganzes verpackt, insbesondere hermetisch dicht verpackt, werden und im Anschluss vorsterilisiert (insbesondere durch Gamma- oder Heißdampfsterilisation) werden, sodass sie nach der Auslieferung sofort in Betrieb genommen werden kann, ohne dass noch Komponenten hinzugefügt oder fixiert werden müssen.

Gemäß einem vorteilhaften Aspekt der Erfindung können sich wenigstens einige der Filtercapsulen hinsichtlich Filterart, Bauart und/oder Baugröße unterscheiden. Unterschiedliche Bauarten sollen auch verschiedene Anschlusstypen und/oder verschiedene Filterbauarten einschließen. Durch die Wahlmöglichkeit der Filtercapsulen lassen sich die einzelnen Filtrationsschritte optimal an die jeweiligen Anforderungen (Durchflussrate, Durchlässigkeit, Filterfläche etc.) anpassen.

Gemäß einer besonders bevorzugten Ausführungsform der Erfindung ist das vorgegebene Raster ein 3x3-Raster für maximal neun Filtercapsulen, und die Leitungen sind so vorkonfiguriert, dass drei oder weniger in einer Reihe des Rasters angeordnete Filtercapsulen einem Leitungszweig zugehörig sind. Auf dieser Basis lassen sich zahlreiche praxisgerechte Aufbauten in kompakter Form realisieren. Beispielsweise können mehrere erfindungsgemäße Einweg-Filtrationsfiltrationseinheiten miteinander kombiniert werden und parallel bzw. in Reihe geschaltet werden. Durch den Aufbau als Raster ist eine modulare Kombination mehrerer Filtrationsvorrichtungen besonders kompakt realisierbar. Generell sind auch andere Raster, beispielsweise 1×2-, 1x3-, 1x4-, 1x5-, 1x6-, 2×2-, 2×3-, 2x4-, 2x5-, 2x6-, 3x4-, 3x5-, 3x6-, 4x4-, 4x5-, 4x6-, 5x5-, 5x6- oder 6x6-Raster denkbar.

Die Vorkonfiguration der erfindungsgemäßen Einweg-Filtrationsvorrichtung kann bereits vorsehen, dass wenigstens zwei parallele Leitungszweige einen gemeinsamen Eingang und/oder einen gemeinsamen Ausgang haben. Ein Zusammenschließen der Leitungszweige vor Inbetriebnahme ist dann nicht mehr notwendig.

Bei bestimmten Aufbauten kann vorgesehen sein, dass ausschließlich Sterilfiltercapsulen oder ausschließlich Vorfiltercapsulen verwendet werden. Besonders vorteilhaft sind aber in der Regel Aufbauten mit einer Kombination von wenigstens einer Sterilfiltercapsule und wenigstens einer Vorfiltercapsule.

Gemäß einem besonders vorteilhaften Aspekt der Erfindung ist in einem nachgeschalteten Leitungszweig eine Kontroll-Filtereinrichtung, vorzugsweise in Form einer Sterilfiltercapsule, angeordnet. Die Kontroll-Filtereinrichtung hat die Aufgabe als "letzte Instanz" sicherzustellen, dass tatsächlich ein steriles Filtrat erzeugt wird. Die Sterilität des Filtrates kann durch einen Integritätstest der Kontroll-Filtereinrichtung bestätigt werden. Das heißt, dass bei bestandenem Integritätstest der Kontroll-Filtereinrichtung sicher davon ausgegangen werden kann, dass diese ordnungsgemäß ihre Funktion erfüllt hat und das Filtrat steril ist.

Bei bestimmten Aufbauten ist es vorteilhaft, in mehreren nachgeschalteten parallelen Leitungszweigen jeweils eine Kontroll-Filtereinrichtung, vorzugsweise in Form einer Sterilfiltercapsule, vorzusehen, insbesondere dann, wenn die Filterfläche nur eines Kontrollfilters sich als nicht ausreichend erweist.

Um ohne zeitaufwendigen Umbau oder sonstige bauliche Modifikation nicht nur die Verwendung einer nachgeschalteten Kontroll-Filtereinrichtung zu ermöglichen, sondern auch deren Integrität unabhängig von den restlichen Filtercapsulen der Einweg-Filtrationsvorrichtung ohne Umbau oder bauliche Modifikation der Vorrichtung zu ermöglichen, sieht die Erfindung vor, dass die Kontroll-Filtereinrichtung Teil einer Baugruppe ist, die für einen separaten Integritätstest der Kontroll-Filtereinrichtung vorgesehen ist und einen sterilisierbaren Luftfilter umfasst. Der Integritätstest der Kontroll-Filtereinrichtung kann bei Verwendung einer solchen strömungstechnisch von der restlichen Einweg-Filtrationsvorrichtung separierbaren Baugruppe je nach Wunsch bereits vor dem bestimmungsgemäßen Filtrationsprozess durchgeführt werden (Pre-Use-Integritätstest), da der sterilisierbare Luftfilter eine Kontamination der noch benötigten Kontroll-Filtereinrichtung verhindert, und/oder erst nach dem bestimmungsgemäßen Filtrationsprozess (Post-Use-Integritätstest).

Insbesondere ist für einen solchen unabhängigen und ohne Umbau durchführbaren Integritätstest der Kontroll-Filtereinrichtung ein Aufbau vorteilhaft, bei dem an einen Zulauf und an einen Ablauf der Kontroll-Filtereinrichtung ein zulaufseitiges erstes Abzweigungsglied bzw. ein ablaufseitiges zweites Abzweigungsglied angeschlossen ist, wobei an eines der beiden freien Enden des ersten Abzweigungsglieds über ein zwischengeschaltetes erstes Absperrventil der Ausgang des sterilisierbaren Luftfilters angeschlossen ist, wobei an eines der beiden freien Enden des zweiten Abzweigungsglieds über ein zwischengeschaltetes zweites Absperrventil optional ein Abfallbehälter angeschlossen ist, wobei mit dem anderen freien Ende des ersten Abzweigungsglieds über ein zwischengeschaltetes drittes Absperrventil die Baugruppe aus der Kontroll-Filtereinrichtung, dem Luftfilter, dem optionalen Abfallbehälter, dem ersten und dem zweiten Absperrventil sowie den beiden Abzweigungsgliedern mit einem Außenanschluss der Einweg-Filtrationsvorrichtung verbunden ist. Unter einem Abzweigungsglied soll hier allgemein jedes Verbindungsleitungsstück verstanden werden, mit dem eine Abzweigung realisiert wird, unabhängig von der konkreten Form oder Bauart des Bauteils. Beispielsweise soll ein T- oderY-förmiges Verbindungsleitungsstück oder ein Mehrwegeventil darunter fallen.

Bei dem oben definierten Aufbau bildet vorzugsweise das verbleibende andere freie Ende des zweiten Abzweigungsglieds einen Filtratausgang der Einweg-Filtrationsvorrichtung, der durch ein viertes Absperrventil verschließbar ist.

Eine leichte Zugänglichkeit der Kontroll-Filtereinrichtung kann dadurch gewährleistet werden, dass die Kontroll-Filtereinrichtung in einem äußeren Leitungszweig angeordnet ist.

Die Kontroll-Filtereinrichtung kann aber auch räumlich abgesetzt außerhalb des vorgegebenen Rasters für die Filtercapsulen angeordnet sein.

Gemäß der Erfindung ist eine zentrale Entlüftung der gesamten Einweg-Filtrationsvorrichtung vorgesehen. Dies wird erreicht durch einen an einem oberen Außenanschluss der Einweg-Filtrationsvorrichtung angeordneten sterilisierbaren Luftfilter zum Entlüften der Einweg-Filtrationsvorrichtung. Außerdem kann über den sterilisierbaren Luftfilter die Integrität der gesamten Einweg-Filtrationsvorrichtung getestet werden.

Ein solcher Entlüftungsfilter muss ausreichend geschützt werden, damit er nicht verblockt. Unter Verblocken soll in diesem Zusammenhang verstanden werden, dass Wasser oder ein sonstiges Medium auf einem die Luftfiltermembran stützenden Vlies eine Art Film bildet. Dadurch gibt es Einschränkungen bzgl. des Luftdurchflusses bzw. ein Luftdurchfluss ist nicht mehr möglich. Dieses Problem besteht insbesondere dann, wenn die Luftfiltermembran und das die Luftfiltermembran stützende Vlies aus gammasterilisierbaren Materialien aufgebaut sind. Die Erfindung sieht mehrere, ggf. miteinander kombinierbare Möglichkeiten vor, den Luftfilter zu schützen bzw. eine mögliche Verblockung vorher sichtbar zu machen, sodass rechtzeitig Maßnahmen ergriffen werden können.

Beispielsweise kann zwischen dem Außenanschluss der Einweg-Filtrationsvorrichtung und dem Eingang des Luftfilters ein hydrophober Schutzfilter zum Schutz des Luftfilters zwischengeschaltet sein, der den Durchtritt von Wasser verhindert.

Insbesondere ist eine Ausbildung des Schutzfilters als Flachfilter vorteilhaft, der ohne Stützvliese ausgebildet ist, wobei die Schutzfiltermembran vorzugsweise aus Polyvinylidenfluorid (PVDF), Polyethylen (PE), hydrophobem Polyethersulfon (PESU) oder Polytetrafluorethylen (PTFE) gebildet ist. Die genannten Materialien gewährleisten, dass eine Verblockung durch Ablagerung von Wasser auf der Membran selbst weitestgehend vermieden ist. Da außerdem absichtlich keine Stützvliese vorgesehen sind, ist eine ansonsten mögliche Filmbildung ausgeschlossen. Damit ist sichergestellt, dass der nachgeschaltete Luftfilter nicht in Kontakt mit Wasser kommt und somit nicht verblocken kann, trotz der dort vorhandenen Stützvliese.

Die nachfolgend beschriebenen erfindungsgemäßen Lösungen zielen auf eine optische Kontrolle des Strömungswegs zwischen dem Außenanschluss der Einweg-Filtrationsvorrichtung und dem Luftfilter ab. Eine optische Kontrollmöglichkeit besteht bei gängigen Filtrationsvorrichtungen nicht, da druckbeständige Schlauchmaterialien nicht bzw. nur bedingt durchsichtig sind. Die hier vorgestellten optischen Kontrollmöglichkeiten dagegen versetzen das Bedienpersonal in die Lage, einen Wassereintritt in den Strömungsweg zum Luftfilter zu erkennen und rechtzeitig Maßnahmen zu ergreifen, bevor der Luftfilter möglicherweise benetzt und in seiner Funktion beeinträchtigt wird.

So ist nach einer ersten erfindungsgemäßen Lösung vorgesehen, dass ein Teil des Strömungswegs zwischen dem Außenanschluss und dem Luftfilter als Schauglas ausgebildet ist. Der allgemein etablierte Begriff Schauglas bezeichnet einen durchsichtigen Röhrenabschnitt und ist bezüglich der Materialauswahl nicht einschränkend zu verstehen. Neben echtem Glas kann auch ein transparenter Kunststoff für die Ausbildung des Röhrenabschnitts verwendet werden.

Eine zweite erfindungsgemäße Lösung besteht darin, in dem Strömungsweg zwischen dem Außenanschluss und dem Luftfilter wenigstens ein Indikator anzuordnen, der auf Wasser reagiert, z. B. durch deutlich sichtbare Farbänderung.

Anstelle eines üblicherweise zur Anbindung des Luftfilters verwendeten gewebeverstärkten und deshalb undurchsichtigen Silikonschlauchs ist nach einer dritten erfindungsgemäßen Lösung vorgesehen, dass ein Teil des Strömungswegs zwischen dem Außenanschluss und dem Luftfilter durch einen wenigstens teilweise durchsichtigen Silikonschlauch gebildet ist, der von einer durchsichtigen stützenden Hülle umgeben ist. Die Hülle sorgt dafür, dass der Silikonschlauch selbst den hohen Prüfdrücken standhält. Dank der Transparenz von Schlauch und Hülle kann der Durchgang von Wasser erkannt werden.

Gemäß einem weiteren besonders vorteilhaften, nicht zur Erfindung gehörenden Aspekt ist ein Einweg-Rohrverteilerstück aus sterilisierbarem Kunststoff vorgesehen, insbesondere für eine erfindungsgemäße Einweg-Filtrationsvorrichtung. Das Einweg-Rohrverteilerstück ist einstückig und weist wenigstens eine Abzweigung und/oder wenigstens eine Abbiegung auf. Unter einer Abzweigung ist hier ein von einer Hauptpfad abgehender Zweig zu verstehen, und unter einer Abbiegung ein sehr stark gekrümmter oder geknickter Verlauf des Hauptpfads, der zu einer signifikanten Richtungsänderung des Strömungsverlaufs führt, z. B. um 90°.

Im Gegensatz zu bekannten Rohrverbindungsteilen, die nur mit zusätzlichen Winkeln und vielen Verbindung, die jeweils wiederum Dichtungen benötigen, zu einer Multiplex-Vorrichtung kombiniert werden können, ermöglicht das Einweg-Rohrverteilerstück einen wesentlich kompakteren Aufbau einer solchen Vorrichtung mit weniger Verbindungen. In der Praxis ergeben sich Bauraumeinsparungen von bis zu 80 % und Verbindungseinsparungen von bis zu 55 %. Außerdem steigt die Prozesssicherheit, und gleichzeitig verringert sich die Komplexität der Montage.

Das Einweg-Rohrverteilerstück ist vorzugsweise so druckstabil ausgelegt, dass es Drücken von mehr als 5 bar, vorzugsweise von mehr als 10 bar standhält.

Vorzugsweise ist in dem Einweg-Rohrverteilerstück eine Berstscheibe integriert. Eine solche Berstscheibe fungiert als Sollbruchstelle und kann grundsätzlich an einer beliebigen Stelle am Einweg-Rohrverteilerstück angeordnet sein, z. B. integriert in einen der Ein-/Ausgänge oder aber auch in eine Seitenwand. Sie dient dazu, die Anlage vor schädlichem Überdruck/Unterdruck zu schützen. Insbesondere kann die Berstscheibe zwischen einer Filtrationsvorrichtung und einem Luftfilter mittels eines T-Stücks oder dergleichen angeordnet sein, wobei ein Abzweig zur Berstscheibe führt.

Die Einweg-Rohrverteilerstücke lassen sich beispielsweise durch TRI-Clamp-Verbindungen, Verschrauben oder Verschweißen druckstabil und verdrehsicher zu einem beliebigen Verbund zusammenfügen. Alternativ können an den offenen Enden der Einweg-Rohrverteilerstücke direkt Sterilkonnektoren angebracht werden.

Gemäß einer bevorzugten Ausführungsform ist wenigstens eine Abbiegung an einem offenen Ende des Einweg-Rohrverteilerstücks gebildet.

Das Einweg-Rohrverteilerstück kann auch zum Entlüften einer gesamten Einweg-Filtrationsvorrichtung genutzt werden, wenn es an oberster Stelle der Vorrichtung angeordnet ist. In diesem Fall ist das Einweg-Rohrverteilerstück mit einem separaten Entlüftungsausgang versehen, der in Einbaulage nach oben weist.

Gemäß einer vorteilhaften Weiterbildung ist das Einweg-Rohrverteilerstück aus einem transparenten Kunststoff gebildet, um beispielsweise einen Entlüftungsprozess besser beobachten zu können.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figur 1 eine perspektivische Ansicht einer Einweg-Filtrationsvorrichtung;
- Figuren 2a bis 2d schematische Darstellungen verschiedener Varianten einer ersten Ausführungsform der erfindungsgemäßen Einweg-Filtrationsvorrichtung ohne Halterung in Seitenansicht bzw. Draufsicht;
- Figuren 3a bis 3c schematische Darstellungen verschiedener Varianten einer zweiten Ausführungsform der erfindungsgemäßen Einweg-Filtrationsvorrichtung ohne Halterung in Seitenansicht bzw. Draufsicht;
- Figuren 4a und 4b schematische Darstellungen verschiedener Varianten einer dritten Ausführungsform der erfindungsgemäßen Einweg-Filtrationsvorrichtung ohne Halterung Draufsicht;
- Figuren 5a bis 5c schematische Darstellungen verschiedener Varianten einer vierten Ausführungsform der erfindungsgemäßen Einweg-Filtrationsvorrichtung ohne Halterung in Seitenansicht bzw. Draufsicht;
- Figur 6 eine schematische Darstellung einer fünften Ausführungsform der erfindungsgemäßen Einweg-Filtrationsvorrichtung ohne Halterung in Draufsicht;
- Figuren 7a bis 7f schematische Darstellungen verschiedener Varianten einer sechsten Ausführungsform der erfindungsgemäßen Einweg-Filtrationsvorrichtung ohne Halterung in Seitenansicht bzw. Draufsicht;
- Figuren 8a bis 8h schematische Darstellungen verschiedener Varianten einer siebten Ausführungsform der erfindungsgemäßen Einweg-Filtrationsvorrichtung ohne Halterung in Seitenansicht bzw. Draufsicht;
- Figuren 9a bis 9c schematische Darstellungen verschiedener Varianten einer achten Ausführungsform der erfindungsgemäßen Einweg-Filtrationsvorrichtung ohne Halterung in Draufsicht;
- Figur 10 eine schematische Darstellung einer neunten Ausführungsform der erfindungsgemäßen Einweg-Filtrationsvorrichtung ohne Halterung in Seitenansicht;
- Figur 11 eine perspektivische Darstellung einer ersten Ausführungsform des nicht erfindungsgemäßen Rohrverteilerstücks;
- Figuren 12a und 12b einen Vergleich zwischen einer Kombination nicht erfindungsgemäßer Rohrverteilerstücke und einer Kombination herkömmlicher Rohrverteilerstücke in Draufsicht; und
- Figur 13 schematisch eine Anordnung des nicht erfindungsgemäßen Rohrverteilerstücks mit Einweg-Behältern.

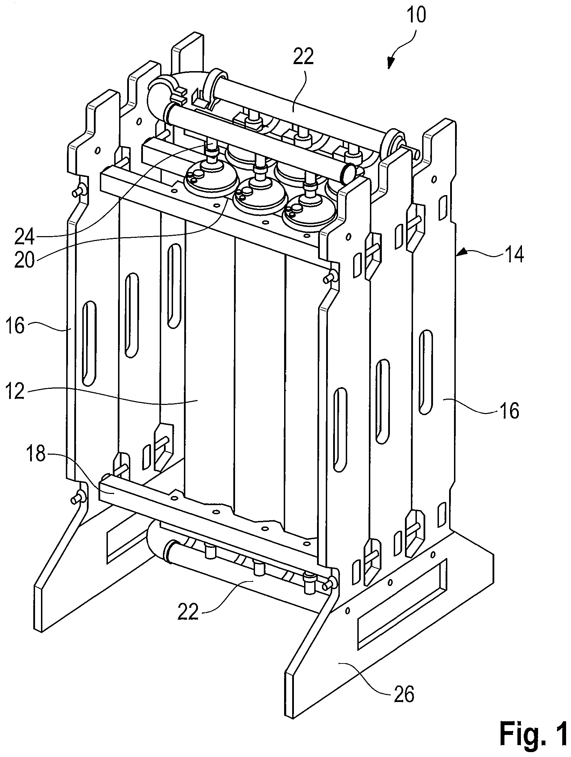

In Figur 1 ist eine Einweg-Filtrationsvorrichtung 10 dargestellt, die der aus der WO 2017/032560 A1 bekannten Vorrichtung ähnlich ist. Eine Mehrzahl von Filtercapsulen 12 wird von einer starren Halterung 14 in einer vorgegebenen Anordnung (Raster) in Position gehalten. Der Begriff "Filtercapsule" ist hier allgemein zu verstehen und soll jegliche eigenständig montierbare Einheit mit wenigstens einem Filter bezeichnen.

Die Halterung 14 umfasst wenigstens zwei gegenüberliegende Seitenwände 16, die durch Querstreben 18 miteinander verbunden sind, wobei die Seitenwände 16 Standfüße 26 aufweisen können. An der Halterung 14 können auch Haltegriffe (nicht gezeigt) vorgesehen sein, um die Handhabung zu vereinfachen. An den Querstreben 18 sind Haltemittel 20 für die einzelnen Filtercapsulen 12 vorgesehen.

Die Filtercapsulen 12 sind vollständig oder zumindest großteils durch starre, druckstabile Rohrleitungen 22 miteinander verbunden. Der Verlauf der Rohrleitungen 22 ist bestimmt durch den vorgesehenen Betrieb der Filtrationsvorrichtung (Parallel- oder Reihenschaltung der Filtercapsulen 12), wobei die Rohrleitungen 22 die notwendigen Abzweigungen 24 zu den einzelnen Filtercapsulen 12 aufweisen. Soweit erforderlich sind die Rohrleitungen 22 an der Halterung 14 befestigt.

Die wesentlichen Bestandteile der starren Halterung 14, die starren Gehäuse der Filtercapsulen 12 und die starren Rohrleitungen 22 sind allesamt vorzugsweise aus demselben Material gebildet. Dieses Material und ggf. weitere Materialien, die bei der Vorrichtung 10 Verwendung finden (z. B. für etwaige flexible Schlauchleitungen), sind sterilisierbar, insbesondere mittels Gammastrahlung, bzw. autoklavierbar. Die Filtrationsvorrichtung 10 kann somit im vormontierten, d. h. anschlussfertigen Zustand sterilisiert und verpackt werden.

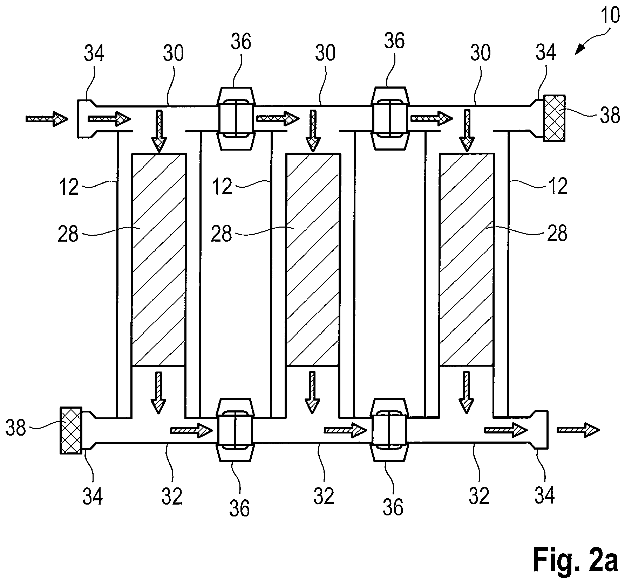

In Figur 2a ist schematisch eine weitere Ausführungsform der Filtrationsvorrichtung 10 dargestellt, jedoch ohne Halterung 14. Anstelle der Rohrleitungen 22 und Abzweigungen 24 sind hier starre, vereinheitlichte Zu- und Ablaufeinrichtungen 30, 32 aus sterilisierbarem, insbesondere gammasterilisierbarem Kunststoff vorgesehen. Genauer gesagt ist für jede Filtercapsule 12, die hier mit den darin aufgenommenen Filterkerzen 28 gezeigt sind, eine eigene Zulaufeinrichtung 30 und eine eigene Ablaufeinrichtung 32 vorgesehen, die auf die stirnseitigen Zulauf- und Ablaufanschlüsse der jeweiligen Filtercapsule 12 abgestimmt sind. Die Zulaufeinrichtung 30 und die Ablaufeinrichtung 32 sind entweder vollständig identisch oder wenigstens großteils identisch aufgebaut. Sowohl die Zulaufeinrichtung 30 als auch die Ablaufeinrichtung 32 weisen jeweils zwei entgegengesetzte Außenanschlüsse 34 auf. Durch geeignete Verbindungsbauteile 36, wie etwa TRI-Clamp-Verbindungen, können mehrere Zu- und Ablaufeinrichtungen 30, 32 unter kontrollierten Bedingungen miteinander verbunden werden. Zwischen den Zu- und Ablaufeinrichtungen 30, 32 befindet sich dazu jeweils eine Dichtung (nicht dargestellt). Alternativ können die Zu- und Ablaufeinrichtungen 30, 32 beispielsweise auch durch Klemmen, Schrauben bzw. Schweißen verbunden werden. Auf diese Weise können beliebig viele Filtercapsulen 12 zusammengefügt werden. Die nicht benötigten Außenanschlüsse 34 werden durch geeignete Verschlüsse 38 abgedichtet. Diese Verschlüsse 38 oder auch Blindkappen werden ebenfalls mittels geeigneter Verbindungsbauteile 36 angebracht. Die Zu- und Ablaufeinrichtungen 30, 32 können einstückig, als Bausatz oder als vorgefertigte Einheit ausgebildet sein. Insbesondere können mehrere Zulaufeinrichtungen 30 und/oder Ablaufeinrichtungen 32 einstückig ausgebildet oder vormontiert sein, bevor sie an den Filtercapsulen 12 angebracht werden.

Insbesondere in der Ausführung mit mehreren zusammenhängenden Zulaufeinrichtungen 30 und/oder Ablaufeinrichtungen 32, die auch als Serienschaltungsdeckel bezeichnet werden können, ergeben sich gegenüber anderen Leitungsverbindungen zahlreiche Vorteile. Insbesondere sind keine oder zumindest weniger große Rohrleitungen 22 und Verbindungsbauteile 36 notwendig. Die Serienschaltungsdeckel ermöglichen das parallele Anströmen mehrerer Filtercapsulen 12 und lassen sich bezüglich der Baugrößen und Bauarten der Filtercapsulen 12 beliebig kombinieren. Insgesamt verhelfen die Serienschaltungsdeckel der Einweg-Filtrationsvorrichtung 10 zu einer kompakteren, kleineren Baugröße, auch weil die Halterung 14 kleiner ausfallen kann. Da bei Verwendung von Serienschaltungsdeckeln weniger Komponenten benötigt werden, ist die Einweg-Filtrationsvorrichtung 10 insgesamt umweltfreundlicher und einfacher zu montieren.

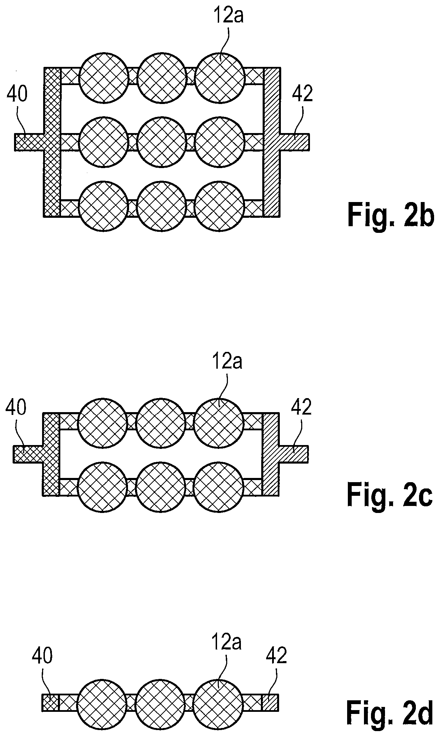

Die Figuren 2b bis 10 zeigen schematisch weitere Beispiele verschiedener Aufbauten (Setups) einer Einweg-Filtrationsvorrichtung 10, wobei für entsprechende Bauteile die gleichen Bezugszeichen verwendet werden.

Bei allen Ausführungsformen sind zumindest die für die wesentlichen Filtrationsprozesse vorgesehenen Filtercapsulen 12 in einem durch die Halterung 14 oder eine andere Halteeinrichtung vorgegebenen Raster angeordnet. Die Filtercapsulen 12 selbst (Filterart, Bauart, Baugröße etc.) und die Verbindungen der Filtercapsulen 12 untereinander mittels der Rohrleitungen 22 oder Zu- und Ablaufeinrichtungen 30, 32 sind frei vorkonfigurierbar. Die Rohrleitungen 22 bzw. Zu- und Ablaufeinrichtungen 30, 32 definieren den Strömungsverlauf durch die einzelnen Filtercapsulen 12, wobei verschiedene Leitungszweige ausgebildet sein können, die nacheinander oder parallel durchströmt werden. Des Weiteren ist allen Ausführungsformen gemein, dass sie von einem Anbieter in sterilisiertem Zustand einsatzbereit an einen Kunden ausgeliefert werden können. Dazu werden die Einweg-Filtrationsvorrichtungen beispielsweise verpackt und anschließend mittels Gammastrahlung sterilisiert.

Es werden zunächst die unterschiedlichen Aufbauten an sich kurz erläutert, bevor dann auf verschiedene Aspekte der Erfindung und bestimmte Anwendungsmöglichkeiten eingegangen wird.

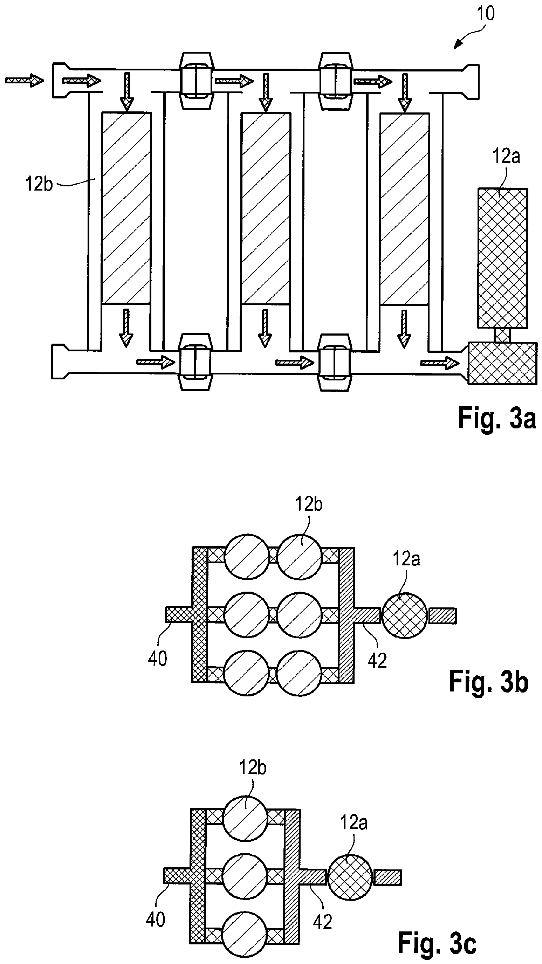

In den Figuren 2b bis 2d sind beispielhaft drei verschiedene Aufbauvarianten in Draufsicht gezeigt, die auf der in Figur 2a gezeigten Ausführungsform der Einweg-Filtrationsvorrichtung 10 basieren mit aufrecht stehenden Filtercapsulen 12, deren Zulaufanschlüsse jeweils am oberen Ende und deren Ablaufanschlüsse jeweils am unteren Ende angeordnet ist. In den Figuren 2b-2d (ebenso wie in den später noch erläuterten Figuren 3b, 3c, 4a, 4b, 5b, 5c, 6, 7b-7f, 8b-8h und 9a-9c) sind die Filtercapsulen 12 nur schematisch ohne Gehäuse gezeigt, um die Anordnung der (verschiedenen) Filtercapsulen 12 zu verdeutlichen.

Bei der Variante nach Figur 2b sind insgesamt neun Filtercapsulen 12 eingesetzt, wobei jeweils drei Filtercapsulen 12 hintereinander in einem Leitungszweig angeordnet sind. Die drei Leitungszweige werden parallel durchströmt und haben einen gemeinsamen Eingang 40 und einen gemeinsamen Ausgang 42. Die Variante nach Figur 2c hat nur zwei parallele Leitungszweige, die Variante nach Figur 2d nur einen. Bei allen Varianten sind Filtercapsulen 12 gleicher Bauart mit gleichen Filtern eingesetzt. Die Filtercapsulen der in den Figuren 2b bis 2d gezeigten Aufbauten sind Filtercapsulen 12 mit Sterilfiltern (nachfolgend als Sterilfiltercapsulen 12a bezeichnet); es können aber auch Filtercapsulen 12 mit Vorfiltern (nachfolgend als Vorfiltercapsulen 12b bezeichnet) vorgesehen sein.

Die in den Figuren 3a bis 3c gezeigten Aufbauten weisen ebenfalls mehrere parallel durchströmte Leitungszweige auf. Allerdings sind hier Vorfiltercapsulen 12b eingesetzt. Außerdem sind bei den Aufbauten nach den Figuren 3b und 3c weniger Filtercapsulen 12 je Leitungszweig vorgesehen, nämlich nur zwei (Figur 3b) bzw. nur eine (Figur 3c). Ergänzend ist zudem eine weitere Filtercapsule vorgesehen, hier eine Sterilfiltercapsule 12a. Diese Sterilfiltercapsule 12a ist in Strömungsrichtung hinter dem zusammengeführten Ausgang 42 der Leitungszweige angeordnet, d. h. sie wird vom Filtrat aller Leitungszweige durchströmt.

In den Ausführungsbeispielen der Figuren 3a bis 3c sind unterschiedliche Bauarten der Filtercapsulen 12 zu sehen. Die Vorfiltercapsulen 12b haben ihren Zulaufanschluss und ihren Ablaufanschluss an entgegengesetzten Enden ihres länglichen Grundkörpers. Die Bauart der Sterilfiltercapsule 12a unterscheidet sich hiervon dahingehend, dass ihr Zulaufanschluss und ihr Ablaufanschluss am selben Ende der Capsule angeordnet sind. Der Grundkörper der Capsule erstreckt sich hier also quer zu den - in der Regel einander gegenüberliegenden - Anschlüssen. Diese Bauart wird als "T-Style" bezeichnet.

Eine Filtercapsule 12 kann auch über nur einen Ein- und über nur einen Ausgang verfügen und in die Leitung vorzugsweise liegend integriert sein. Diese Bauart wird als "In-Line" bezeichnet.

In-Line- und T-Style-Filtercapsulen haben gemeinsam, dass mit ihnen eine Verschaltung mehrerer Filtercapsulen in Reihe erfolgen kann. Bei der Bauart mit Serienschaltungsdeckel werden mehrere Filtercapsulen parallel angeströmt.

Die Sterilfiltercapsule 12a hat bei den Aufbauten der Figuren 3a bis 3c jeweils die Funktion eines Kontrollfilters, worauf später noch genauer eingegangen wird.

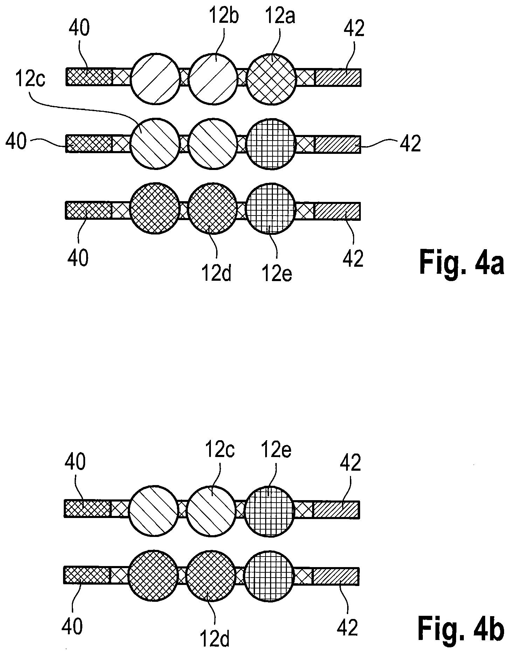

Bei den in den Figuren 4a und 4b gezeigten Aufbauten hat die Einweg-Filtrationsvorrichtung 10 mehrere Leitungszweige, die insoweit unabhängig voneinander sind. Die einzelnen Leitungszweige haben also separate Eingänge und separate Ausgänge. Dennoch sind alle Leitungszweige feste Bestandteile der vorkonfigurierten Einweg-Filtrationsvorrichtung 10. Die Leitungszweige weisen verschiedene Typen von Filtercapsulen 12a, 12b, 12c, 12d und 12e auf, wobei sich die Filtercapsulen hinsichtlich Filterart (Vorfilter, Sterilflter etc.), Bauart (Serienschaltungsdeckel, In-Line, T-Style etc.) und Baugröße unterscheiden können.

Ein Anbieter kann durch einen solchen modularen Aufbau verschiedene Prozesse an sich oder auch einen, zwei oder mehrere Prozessschritte bereitstellen. Somit kann ein Kunde für die Durchführung verschiedener Prozesse oder Teilprozesse die verschiedenen Aufbauten innerhalb einer einzigen Einweg-Filtrationsvorrichtung 10 nutzen. Dabei ist die Filtrationsvorrichtung 10 besonders platzsparend und deren Installation besonders zeitsparend.

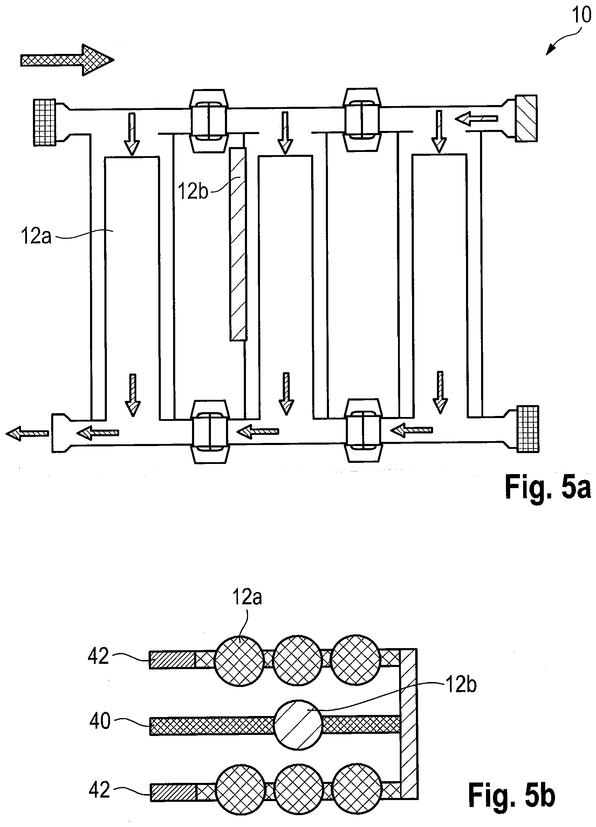

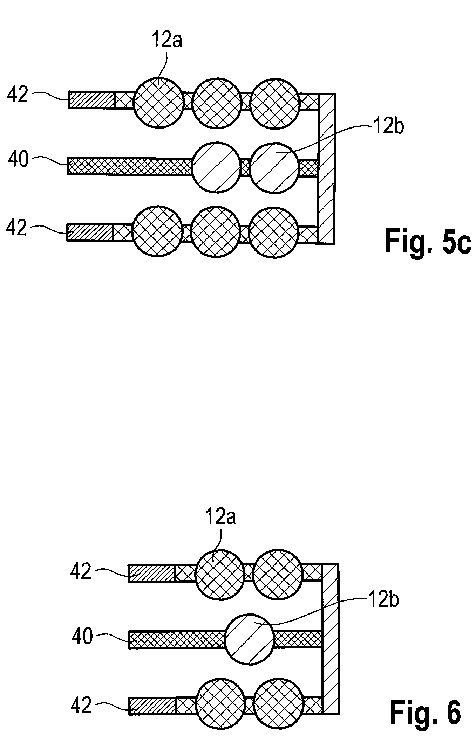

Die Aufbauten der Figuren 5a bis 5c sowie der Figur 6 weisen eine Kombination aus mehreren Sterilfiltercapsulen 12a und einer oder mehreren vorgeschalteten Vorfiltercapsulen 12b auf. Die Vorfiltercapsulen 12b sind in einem Eingangsleitungszweig angeordnet, von dem zwei parallel durchströmte Leitungszweige mit den Sterilfiltern 12a abzweigen. Die Vorfiltercapsulen 12b sind hier als T-Style-Capsulen ausgeführt und mit ihren Zulauf- und Ablaufanschlüssen in den (hier oberen) Eingangsleitungszweig integriert, sodass das entgegengesetzte Ende der Vorfiltercapsulen 12b nach unten hängt. Es ist aber auch eine liegende Anordnung mit In-Line-Capsulen möglich.

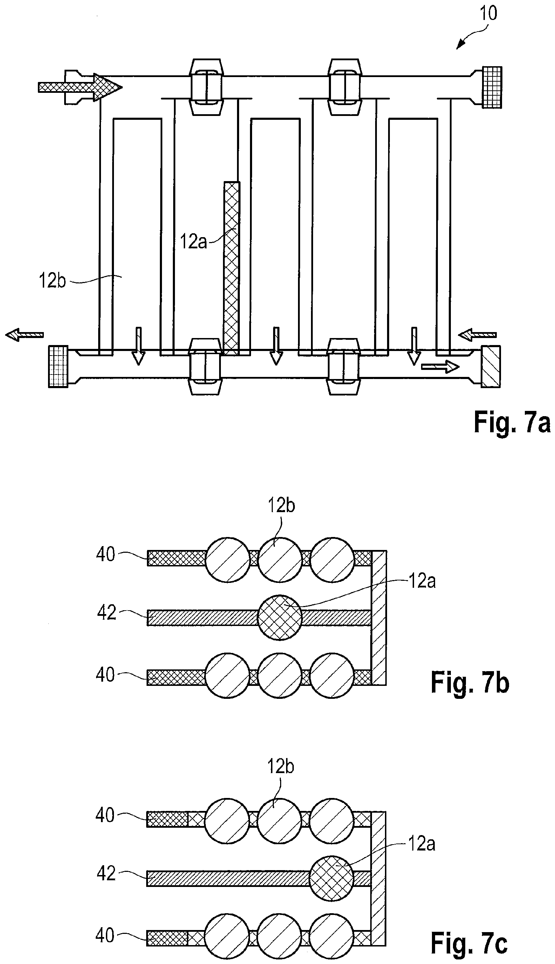

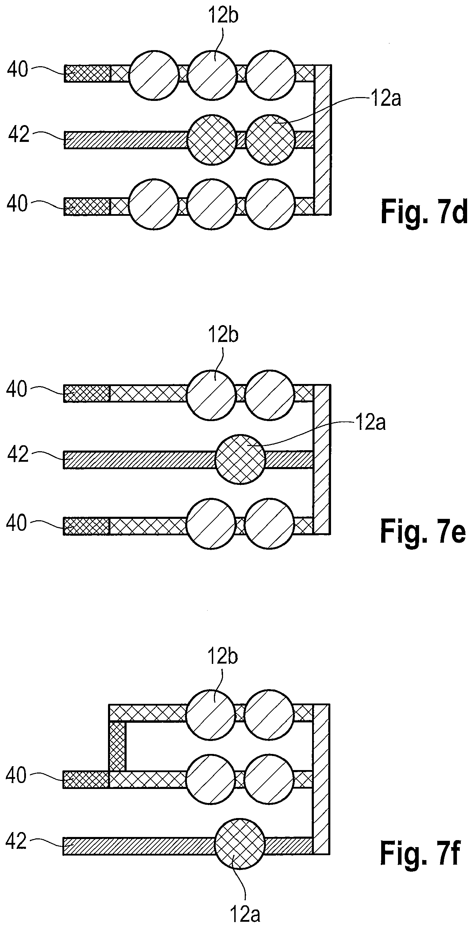

Die in den Figuren 7a bis 7f gezeigten Aufbauten unterscheiden sich von denen der Figuren 5a bis 6b durch die Anordnung der Eingänge 40 und Ausgänge 42 und die Anzahl der Vorfilter 12b und der Sterilfilter 12a. Die Sterilfiltercapsulen 12a sind den in parallelen Eingangsleitungszweigen angeordneten Vorfiltercapsulen 12b nachgeschaltet. Die Sterilfiltercapsulen 12a sind "stehend" angeordnet, d. h. deren Zulauf- und Ablaufanschlüsse sind in den unteren Eingangsleitungszweig integriert, sodass das entgegengesetzte Ende der Sterilfiltercapsulen 12a nach oben ragt. Es ist aber auch eine liegende Anordnung mit In-Line-Filtercapsulen 12 möglich.

Während bei den Aufbauten der Figuren 7a bis 7e die Sterilfiltercapsulen 12a in einem mittleren Leitungszweig angeordnet sind, ist die Sterilfiltercapsule 12a beim Aufbau nach Figur 7f in einem äußeren, leicht zugänglichen Leitungszweig angeordnet.

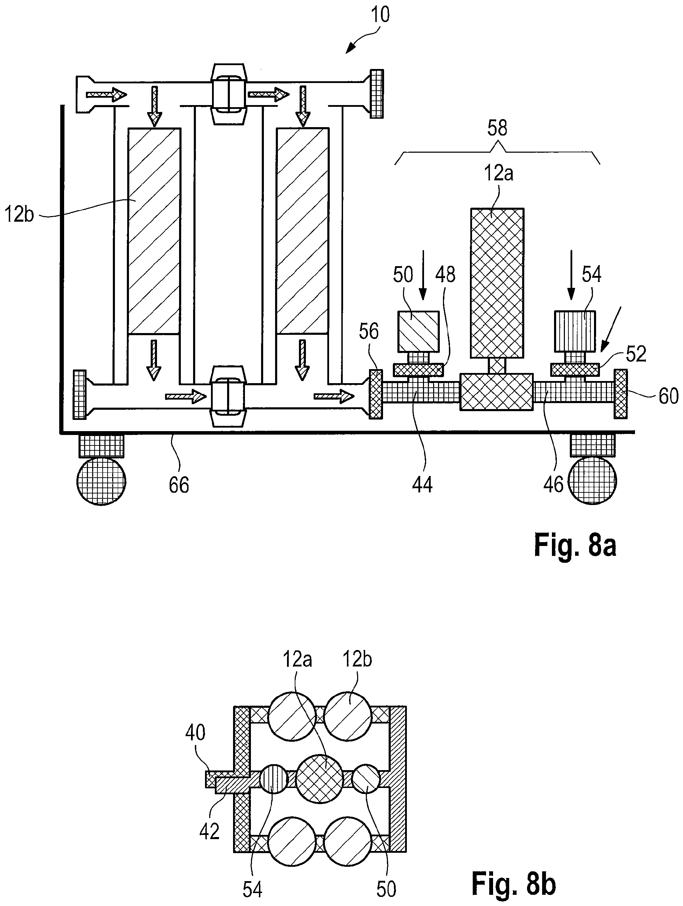

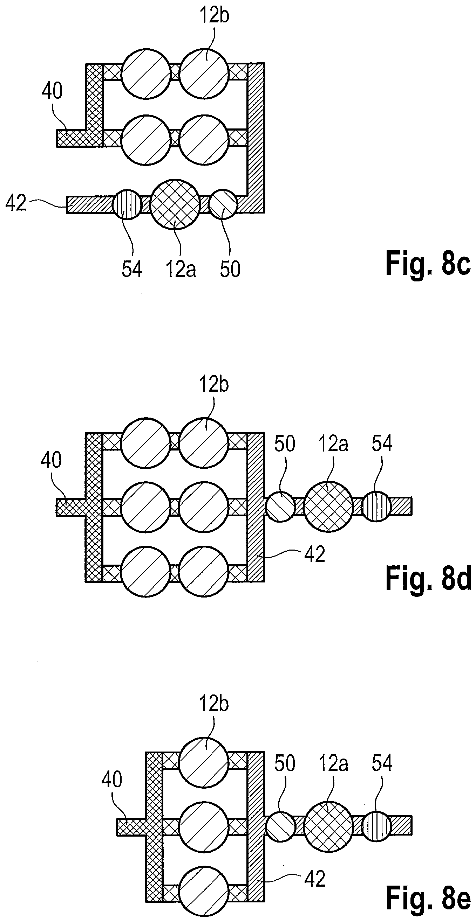

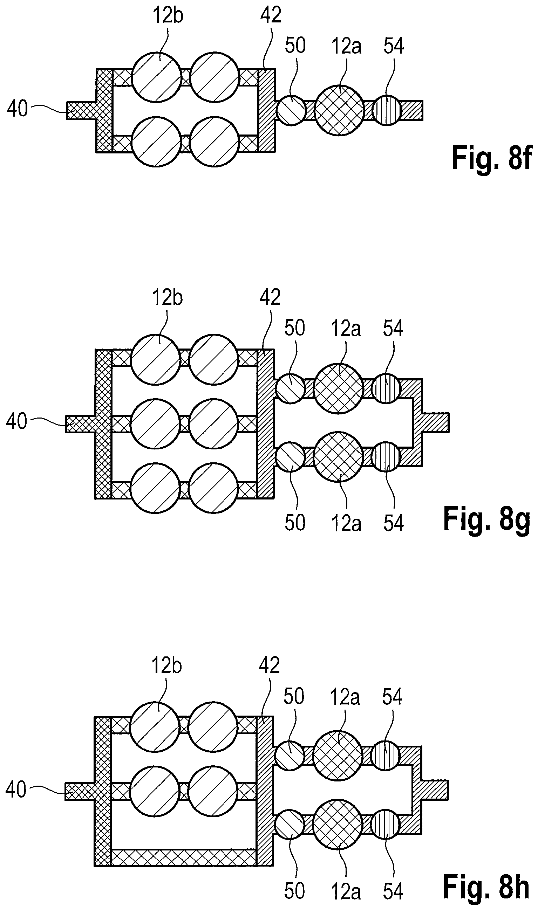

In den Figuren 8a bis 8h sind verschiedene Aufbauten gezeigt, bei denen wenigstens eine Sterilfiltercapsule 12a in Form einer T-Style oder In-Line-Capsule vorgesehen ist, die als Kontroll-Filtereinrichtung (nachfolgend der Einfachheit halber als Kontrollfilter bezeichnet) fungiert, wie später noch erläutert wird. An den Zulauf und an den Ablauf dieses Kontrollfilters ist ein zulaufseitiges erstes Abzweigungsglied 44 bzw. ein ablaufseitiges zweites Abzweigungsglied 46 angeschlossen. An eines der beiden freien Enden des ersten Abzweigungsglieds 44 ist über ein zwischengeschaltetes erstes Absperrventil 48 der Ausgang eines sterilisierbaren, insbesondere gammasterilisierbaren Luftfilters 50 angeschlossen. An eines der beiden freien Enden des zweiten Abzweigungsglieds 46 ist über ein zwischengeschaltetes zweites Absperrventil 52 ein Abfallbehälter 54 (waste bag) angeschlossen. Mit dem anderen freien Ende des ersten Abzweigungsglieds 44 ist über ein zwischengeschaltetes drittes Absperrventil 56 die gesamte, nachfolgend als Einweg-Kontrolleinheit 58 bezeichnete Baugruppe aus Kontrollfilter, Luftfilter 50, Abfallbehälter 54, den beiden Abzweigungsgliedern 44 und 46 und dem ersten und dem zweiten Absperrventil 48, 52 mit einem Ausgangsanschluss (Außenanschluss 34) der restlichen Einweg-Filtrationsvorrichtung 10 verbunden. Das andere freie Ende des zweiten Abzweigungsglieds 46 stellt letztlich den Filtratausgang der Einweg-Filtrationsvorrichtung 10 dar. Der Filtratausgang ist durch ein viertes Absperrventil 60 verschließbar, das ebenfalls Bestandteil der Einweg-Kontrolleinheit 58 ist.

Bei dem in Figur 8b gezeigten Aufbau ist die Einweg-Kontrolleinheit 58 zwischen zwei parallel durchströmten Leitungszweigen mit Vorfiltern 12b angeordnet. Dagegen ist bei dem in Figur 8c gezeigten Aufbau die Einweg-Kontrolleinheit 58 in einem äußeren, leicht zugänglichen Leitungszweig der Einweg-Filtrationsvorrichtung 10 angeordnet. Beide Aufbauten sind vergleichsweise platzsparend.

Die Figuren 8d bis 8h zeigen verschiedene weitere Aufbauten, bei denen eine oder mehrere parallel angeordnete Einweg-Kontrolleinheiten 58 an einen bzw. mehrere (gemeinsame) Ausgänge 42 der vorgeschalteten Leitungszweige der Einweg-Filtrationsvorrichtung 10 angehängt sind. Das bedeutet, dass die Einweg-Kontrolleinheit 58 jeweils außerhalb des durch die Halterung 14 der Einweg-Filtrationsvorrichtung 10 vorgegebenen Rasters für die Filtercapsulen 12 angeordnet sind, wobei die Kontrolleinheit 58 dennoch Bestandteil der Einweg-Filtrationsvorrichtung 10 ist..

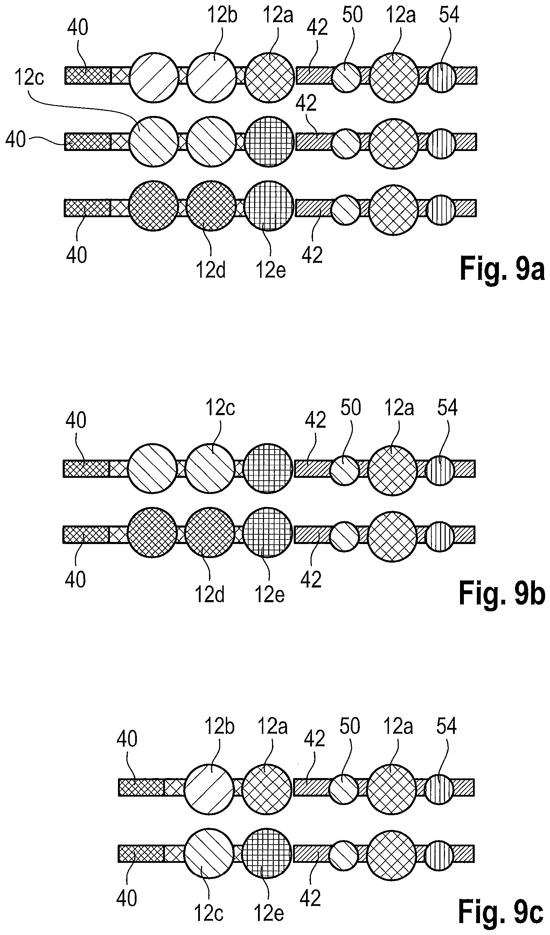

Die Figuren 9a bis 9c zeigen weitere Aufbauten mit mehreren voneinander unabhängigen Leitungszweigen (ähnlich wie in den Figuren 4a und 4b), die alle feste Bestandteile der vorkonfigurierten Einweg-Filtrationsvorrichtung 10 sind. Die Leitungszweige weisen verschiedene Typen von Filtercapsulen 12a, 12b, 12c, 12d und 12e auf, wobei sich die Filtercapsulen hinsichtlich Filterart (Vorfilter, Sterilflter etc.), Bauart (In-Line, T-Style etc.) und Baugröße unterscheiden können. An jeden Leitungszweig ist eine separate Einweg-Kontrolleinheit 58 angehängt. Dadurch kann ein Anwender den Kontrollfilter jeder separaten Leitung entsprechend prüfen.

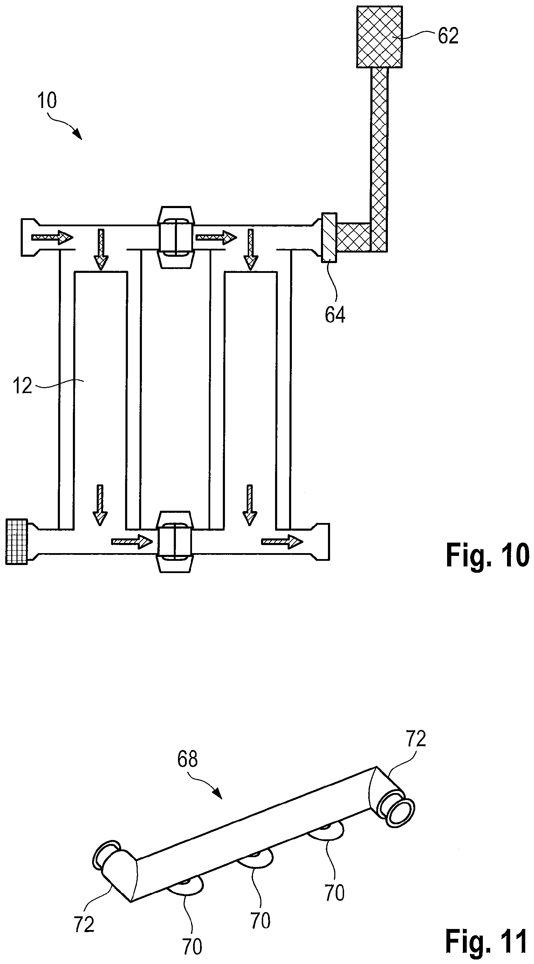

In Figur 10 ist eine Einweg-Filtrationsvorrichtung 10 ohne Halterung 14 dargestellt, bei der an einem oberen Außenanschluss 34 ein sterilisierbarer, insbesondere gammasterilisierbarer Luftfilter 62 angeschlossen ist. Der Außenanschluss 34 für den Luftfilter 62 muss nicht zwingend an einer Zu- oder Ablaufeinrichtung 30, 32 gebildet sein. Der Luftfilter 62 kann alleine das Entlüften der gesamten Einweg-Filtrationsvorrichtung 10 bewerkstelligen, oder es sind mehrere solcher Luftfilter 62 an verschiedenen Außenanschlüssen 34 vorgesehen. Zwischen dem Außenanschluss 34 und dem Eingang des Luftfilters 62 ist ein eigener Schutzfilter 64 zum Schutz des Luftfilters 62 zwischengeschaltet.

Die oben beschriebenen Aufbauten stellen nur eine Auswahl einer Vielzahl von möglichen Aufbauten dar. Viele weitere Kombinationsmöglichkeiten sind innerhalb der Einweg-Filtrationsvorrichtung 10 möglich.

Nachfolgend werden besondere Aspekte der Erfindung und bestimmte Anwendungsmöglichkeiten näher erläutert.

Die Einweg-Filtrationsvorrichtung 10 ist vor der Sterilisation und der Auslieferung an einen Kunden beliebig vorkonfigurierbar. Es können verschiedene Arten von Filtercapsulen 12 (Filterart, Bauart, Baugröße etc.) an den die durch die Halterung 14 vorgegebenen Stellen eingesetzt werden. Dabei ist zu berücksichtigen, dass auch die Halterung 14 selbst beliebig konfigurierbar ist. Beispielsweise kann sie ein 3x3-Raster ebenso wie ein 2x3-Raster oder beliebige andere Raster für die Filtercapsulen 12 vorgeben. Es sind viele Kombinationen von Filtercapsulen 12 möglich, insbesondere von Vorfiltercapsulen 12b und Sterilfiltercapsulen 12a oder, wie etwa bei einer redundanten Sterilfiltration, mit einer Kombination von mehreren Sterilfiltercapsulen 12a mit gleicher Porosität, z. B. 0,2 µm, oder unterschiedliche Porosität, z. B. 0,2 µm und 0,1 µm, innerhalb derselben Einweg-Filtrationsvorrichtung 10.

Die Filtercapsulen 12 werden an geeignete Haltemittel 20 geklemmt, geschweißt, geschraubt, geklebt oder auf sonstige Weise stabil befestigt.

Mittels der Rohrleitungen 22 und/oder der Zu- und Ablaufeinrichtungen 30, 32 können verschiedene Strömungspfade mit einem oder mehreren Leitungszweigen definiert werden, die nacheinander oder parallel durchströmt werden können. Je nach Bedarf können innerhalb der Einweg-Filtrationsvorrichtung 10 miteinander verbundene Leitungszweige oder getrennte, voneinander unabhängige Strömungswege (Prozessstränge) durch die Filtercapsulen 12 realisiert werden.

Auf diese Weise lassen sich innerhalb der kompakten Einweg-Filtrationsvorrichtung 10 platzsparend stabile, komplett vorsterilisierbare Setups für verschiedene Prozesse aufbauen.

Nachdem in der Halterung 14 ein mit dem Kunden vereinbartes Setup aufgebaut worden ist, wird die gesamte Einweg-Filtrationsvorrichtung 10 verpackt, sterilisiert und als betriebsbereite Einheit ausgeliefert. Der Kunde kann die Einweg-Filtrationsvorrichtung 10 sofort nach dem Auspacken benutzen, da für den von ihm gewünschten Filtrationsprozess keine weiteren Komponenten in die Einweg-Filtrationsvorrichtung 10 integriert werden müssen.

Wie in Figur 8a exemplarisch gezeigt kann die gesamte Einweg-Filtrationsvorrichtung 10 für eine bessere lokale Beweglichkeit und Handhabung auf einem Wagen 66 oder auf einer anderen beweglichen Einrichtung angeordnet werden. Wie bereits erwähnt kann die Einweg-Filtrationsvorrichtung 10 alternativ oder zusätzlich an der Halterung 14 auch einen Griff aufweisen.

Die in den Ausführungsformen der Figuren 3a bis 3c und 8a bis 8h als Kontrollfilter verwendete Sterilfiltercapsule 12a hat die Aufgabe, als "letzte Instanz" sicherzustellen, dass tatsächlich ein steriles Filtrat erzeugt wurde. Die Sterilität des Filtrates wird durch das Bestehen eines Integritätstests des Kontrollfilters bestätigt.

Der Kontrollfilter ist aus folgendem Grund stromabwärts der für den eigentlichen Filtrationsprozess vorgesehenen Filtercapsulen 12 der Einweg-Filtrationsvorrichtung 10 angeordnet: Wenn die Einweg-Filtrationsvorrichtung 10 auf Integrität getestet wird, kann aus einer erwarteten Gesamtdiffusion (ml/min), die sich aus den Sollwerten aller Filtercapsulen 12 bzw. der darin angeordneten einzelnen Filterkerzen 28 ergibt, nicht zwingend darauf geschlossen werden, dass alle Filtercapsulen 12 bzw. Filterkerzen 28, ihren jeweiligen Grenzwert nicht überschreiten. Beispielsweise kann eine nicht integere (defekte) Filterkerze 28 ihren Grenzwert überschreiten und eine oder mehrere andere ihren Grenzwert aber unterschreiten, sodass sich die Werte insoweit ausgleichen, dass in der Summe der Erwartungswert für die gesamte Einweg-Filtrationsvorrichtung 10 nicht überschritten wird. Dennoch könnte die defekte Filterkerze 28 z. B. Bakterien durchlassen, was dazu führen würde, dass das Filtrat insgesamt nicht steril wäre. Deshalb ist nach der regulären Filtration eine separate Kontrollfiltration mittels einer separaten Filtercapsule 12, insbesondere einer Sterilfiltercapsule 12a vorgesehen, durch die am Ende das gesamte Filtrat durchläuft. Dieser einzelne Kontrollfilter lässt sich als separate Einheit ohne großen Aufwand zuverlässig auf Integrität testen. Bei bestandenem Integritätstest des Kontrollfilters kann davon ausgegangen werden, dass das insgesamt gewonnene Filtrat tatsächlich steril ist.

Alternativ oder zusätzlich zur Diffusionsmessung kann im Rahmen eines Integritätstests des Kontrollfilters auch der sogenannte Bubble-Point bestimmt werden. Die Poren des Filters (bei Sterilfiltration z. B. 0,2 µm) werden mit einem Benetzungsmedium befüllt, was am einfachsten durch Durchspülen der Filtercapsule12a mit Druck gelingt. Für den Test wird der Filter, bevorzugt von der Anströmseite, langsam mit Druck beaufschlagt. Um das Benetzungsmedium aus der Pore zu verdrängen, ist eine Kraft (Druckdifferenz der beiden Filterseiten) nötig. Diese Druckdifferenz ist vom Porendurchmesser abhängig. Der Druck auf den Filter wird erhöht, und sobald ein kontinuierlicher Luftblasenaustritt zu erkennen ist, wird der Druck am Prüfgerät, beispielsweise an einem Manometer abgelesen. Da Oberflächenspannung, Benetzungswinkel und Druckdifferenz bekannt sind, kann theoretisch die größte Pore der Membran errechnet und somit deren Qualität bestimmt werden. Üblicherweise wird aber einfach der abgelesene Druck mit dem vom Filterhersteller angegebenen Grenzwert verglichen.

Je nach Aufbau können auch zwei oder mehrere Kontrollfilter parallel eingesetzt werden.

Der Kontrollfilter ist in die Einweg-Filtrationsvorrichtung 10 integriert, d. h. er ist fester Bestandteil der bereits vor der Auslieferung sterilisierten Einweg-Filtrationsvorrichtung 10. Der Kontrollfilter muss also nicht nachträglich über Schlauchsysteme nachgeschaltet werden, was einen zusätzlichen Material- sowie Zeitaufwand zum Aufbau einer sicheren und stabilen Verbindung bedeuten würde. Ein zusätzlicher Halter oder dergleichen zur Fixierung des Kontrollfilters wird nicht benötigt, da der Kontrollfilter als fixierte Komponente mit ausgeliefert wird.

Bei den in den Figuren 8a bis 9c gezeigten Aufbauten liegt der besondere Vorteil darin, dass der in die Einweg-Filtrationsvorrichtung 10 integrierte Kontrollfilter (Sterilfiltercapsule 12a) auf Integrität getestet werden kann, ohne dass ein Umbau oder eine Demontage von Komponenten der Einweg-Filtrationsvorrichtung 10 notwendig ist. Der Integritätstest des Kontrollfilters kann unmittelbar vor der bestimmungsgemäßen Benutzung der Einweg-Filtrationsvorrichtung 10 (Pre-Use-Integritätstest) und/oder nach der Benutzung (Post-Use-Integritätstest) durchgeführt werden.

Beim Pre-Use-Integritätstest werden das erste Absperrventil 48 zum sterilen Luftfilter 50 und das vierte Absperrventil 60 am Filtratausgang der Einweg-Kontrolleinheit 58 geschlossen. Das zweite Absperrventil 52 zum Abfallbehälter 54 wird geöffnet. Nach dem Öffnen des dritten Absperrventils 56 werden die Filter der Filtercapsulen 12 einschließlich des Kontrollfilters mit einem geeigneten flüssigen Medium benetzt. Überschüssiges Benetzungsmedium gelangt in den Abfallbehälter 54. Nach der Benetzung der Filter wird das dritte Absperrventil 56 wieder geschlossen, sodass die Einweg-Kontrolleinheit 58 strömungstechnisch vom Rest der Einweg-Filtrationsvorrichtung 10 separiert ist. Das erste Absperrventil 48 zum Luftfilter 50 wird geöffnet. An den Eingang des Luftfilters 50 wird nun ein Prüfgerät angeschlossen, mit dem die für den Integritätstest vorgesehenen Prüfungen, insbesondere die Diffusionsmessung und/oder die Bestimmung des Bubble-Points des Kontrollfilters durchgeführt werden. Hierbei wird durch den sterilen Luftfilter 50 sterilisierte Luft in die Einweg-Kontrolleinheit 58 gepumpt. Die während des Integritätstest durch den Kontrollfilter gedrückte Flüssigkeit gelangt in den Abfallbehälter 54 und kann entsorgt werden, nachdem das zweite Absperrventil 52 geschlossen wurde. Nach Abschluss des Pre-Use-Integritätstests wird das erste Absperrventil 48 zum Luftfilter 50 geschlossen und das dritte Absperrventil 56 und das vierte Absperrventil 60 werden geöffnet. Die Einweg-Filtrationsvorrichtung 10 einschließlich des Kontrollfilters steht nun zum bestimmungsgemäßen Gebrauch bereit.

Alternativ oder zusätzlich zum Pre-Use-Integritätstest kann nach der Durchführung des Filtrationsprozesses auf folgende Weise ein Post-Use-Integritätstest durchgeführt werden, um zu überprüfen, ob der Kontrollfilter fehlerhaft ist. Nach Beendigung des Filtrationsprozesses werden das dritte Absperrventil 56 und das vierte Absperrventil 60 am Filtratausgang geschlossen. Das erste Absperrventil 48 zum Luftfilter 50 und das zweite Absperrventil 52 zum Abfallbehälter 54 werden geöffnet. Nun kann wie oben bereits beschrieben der Integritätstest mithilfe eines am Eingang des Luftfilters 50 angeschlossenen Prüfgeräts durchgeführt werden. Alternativ kann vor dem Test das Filtrat diskonnektiert und ein Abfallbehälter 54 (sofern überhaupt erforderlich) auch am vierten Absperrventil 60 angeschlossen werden. In diesem Fall muss für die Durchführung des Post-Use-Integritätstests das zweite Absperrventil 52 geschlossen und das vierte Absperrventil 60 geöffnet werden. Nach Beendigung des Integritätstests werden alle offenen Ventile geschlossen, damit beim Entsorgen der gesamten Einweg-Filtrationsvorrichtung 10 keine Flüssigkeit unkontrolliert austreten kann.

Bei einem erst nach dem bestimmungsgemäßen Gebrauch einer Einweg-Filtrationsvorrichtung nach dem stand der Technik über zusätzliche Schlauchsysteme nachgeschaltetem Kontrollfilter ist es grundsätzlich nicht sinnvoll, einen Pre-Use-Integritätstest durchzuführen, da das sterile System diskonnektiert werden müsste und dadurch die komplette Einweg-Filtrationsvorrichtung 10 unsteril würde. Ein Post-Use-Integritätstest kann nur durchgeführt werden, nachdem der Kontrollfilter von dem der Einweg-Filtrationsvorrichtung 10 nachgeschalteten Schlauchsystem getrennt wird. Dies ist mit zusätzlichem Zeit- und Arbeitsaufwand verbunden.

Sollte die Filterfläche eines Kontrollfilters im Anschluss an die Vorfiltercapsulen 12b und/oder Sterilfiltercapsulen 12a nicht ausreichen, so ist es möglich, auch zwei oder mehr parallel durchströmte Kontrollfilter in der Einweg-Filtrationsvorrichtung 10 nachzuschalten, wie beispielhaft in den Figuren 8g und 8h gezeigt ist. Jeder der parallel verbundenen Kontrollfilter kann einzeln einem Pre-Use-Integritätstest und/oder einem Post-Use-Integritätstest unterzogen werden. Entsprechendes gilt für die in den Figuren 9a bis 9c gezeigten und vergleichbaren Aufbauten, bei denen mehrere Kontrollfilter vorgesehen sind, welche jeweils an einen einzelnen Prozessstrang der Einweg-Filtrationsvorrichtung 10 angehängt sind.

Bei der Einweg-Filtrationsvorrichtung 10 ist, unabhängig von deren konkretem Aufbau, üblicherweise an einem der oberen Außenanschlüsse 34 ein sterilisierbarer, insbesondere gammasterilisierbarer Luftfilter 62 zum Entlüften vorgesehen, wie schematisch in Figur 10 gezeigt. Obwohl grundsätzlich auch mehrere solcher Luftfilter 62 vorgesehen sein können, wird der Einfachheit halber nachfolgend von nur einem zentralen Luftfilter 62 für alle Filtercapsulen 12 ausgegangen, die am bestimmungsgemäßen Filtrationsprozess beteiligt sind (also ausgenommen eines etwaigen Kontrollfilters, der in einer Einweg-Kontrolleinheit 58 zusammen mit einem eigenen Luftfilter 50 angeordnet ist). Der Luftfilter 62 ist typischerweise als Filterkerze mit einer zwischen zwei Stützvlies-Lagen angeordneten plissierten Luftfiltermembran ausgebildet.

Grundsätzlich muss beim Befüllen der Einweg-Filtrationsvorrichtung 10 mit Wasser oder einer anderen Flüssigkeit beachtet werden, dass Flüssigkeit an den Luftfilter 62 gelangen kann. Diese Flüssigkeit kann auf dem Stützvlies eine Art Film bilden und dadurch den Luftfilter 62 verblocken. Durch das Verblocken des Luftfilters 62 ist kein bzw. nur noch ein geringer Luftdurchsatz möglich, womit die grundlegende Funktion des Luftfilters 62 eingeschränkt ist. Außerdem ist zu berücksichtigen, dass der Luftfilter 62 auch dafür vorgesehen ist, vor Beginn oder nach Beendigung des Filtrationsprozesses die beteiligten Filtercapsulen 12 bzw. die gesamte Einweg-Filtrationsvorrichtung 10 auf Integrität zu testen. Durch das Verblocken des Luftfilters können bei einem solchen Test Probleme auftreten.

Bei bekannten Filtrationsvorrichtungen kommt erschwerend hinzu, dass der Luftfilter typischerweise durch einen gewebeverstärktem Schlauch an die Einweg-Filtrationsvorrichtung 10 angebunden ist. Die Verwendung eines gewebeverstärkten Schlauches ist begründet durch die teils sehr hohen Prüfdrücke der Flüssigfilter bzw. die sehr hohen Prozessdrücke. Wegen des gewebeverstärkten Schlauchs ist aber für den Operator optisch nicht zu erkennen, ob sich Wasser oder andere Filtrationslösungen zum Luftfilter hinbewegen. Ein rechtzeitiges Abstellen des Filtrationsprozesses oder Abschotten des Luftfilters mithilfe eines Ventils oder dergleichen ist somit nicht ohne Weiteres möglich.

Im Folgenden werden mehrere Möglichkeiten vorgestellt, die Problematik eines durch Wasser oder Filtermedium blockierten Luftfilters 62 zu vermeiden. Die einzelnen Lösungsaspekte lassen sich auch beliebig miteinander kombinieren.

Gemäß einem ersten Lösungsaspekt ist zwischen dem Außenanschluss 34 und dem Eingang des Luftfilters 62 ein Schutzfilter 64 zwischengeschaltet, wie in Figur 10 gezeigt. Der Schutzfilter 64 ist als Flachfilter ohne Stützvliese oder dergleichen ausgebildet, wobei die Luftfiltermembran aus Polyvinylidenfluorid (PVDF), Polyethylen (PE), hydrophobem Polyethersulfon (PESU) oder Polytetrafluorethylen (PTFE) gebildet sein kann. Da der Schutzfilter 64 keine Stützvliese aufweist, ist die Gefahr einer Verblockung minimiert. Der Operator muss also nicht mehr auf eine mögliche Verblockung achten.

Gemäß einem zweiten Lösungsaspekt ist ein Teil des Strömungswegs zwischen dem Außenanschluss 34 und dem Luftfilter 62 als stabiles Schauglas ausgebildet. Wie bereits eingangs erwähnt, soll unter einem Schauglas ein durchsichtiger Röhrenabschnitt verstanden werden, der insbesondere aus Glas oder einem transparenten Kunststoff gebildet sein kann. Das Schauglas ermöglicht eine optische Kontrolle durch den Operator, der Maßnahmen ergreifen kann, sobald er erkennt, dass Wasser oder ein sonstiges Medium zum Luftfilter 62 hochsteigt. Dabei bestimmen der eingestellte Volumenstrom, die Länge der Leitung zum Luftfilter 62 und die Position des Schauglases in dieser Leitung die Zeitspanne, die dem Operator zum Ergreifen von Maßnahmen verbleiben, bevor das Wasser bzw. Medium den Luftfilter 62 erreicht.

Gemäß einem dritten Lösungsaspekt ist in dem Strömungsweg zwischen dem Außenanschluss 34 und dem Luftfilter 62 wenigstens ein Indikator angeordnet, der auf Wasser reagiert. Solche Wasser-Kontakt-Indikatoren sind als Klebebänder, die sich bei Berührung mit Wasser rot verfärben, oder auf Basis von Blaugel (Kieselgel) erhältlich, das sich blassrosa verfärbt. Ein solcher Indikator ermöglicht eine optische Kontrolle durch den Operator, der ggf. geeignete Maßnahmen ergreifen kann.

Gemäß einem vierten Lösungsaspekt ist anstelle des gewebeverstärkten Silikonschlauchs ein wenigstens teilweise durchsichtiger Standard-Silikonschlauch vorgesehen, der von einer ebenfalls transparenten stützenden Hülle umgeben ist. Als Hülle kommen z. B. reißfeste Folien, eine stabile Ummantelung oder ein eng anliegendes Rohr in Betracht. Die Hülle verhindert, dass der Standard-Silikonschlauch, der eigentlich nur für geringe Drücke zugelassen ist, bei hohen Drücken birst. Aufgrund der transparenten Materialien von Schlauch und Hülle ist dennoch eine optische Kontrolle durch den Operator möglich.

In Figur 11 ist beispielhaft ein starres Kunststoff-Rohrverteilerstück 68 gezeigt, das bei der Einweg-Filtrationsvorrichtung 10, aber auch bei anderen Einweg-Vorrichtungen, insbesondere im biopharmazeutischen Bereich, Anwendung finden kann.

Hauptzweck des Rohrverteilerstücks 68 ist es, eine Strömungsverbindung zwischen zwei oder eine Strömungsverteilung auf mehrere Einweg-Komponenten herzustellen. Das grundsätzlich rohrförmige Rohrverteilerstück 68 ersetzt insbesondere herkömmliche Schlauchverbindungen und besteht aus einem sterilisierbaren, insbesondere gammasterilisierbaren Kunststoff und ist autoklavierbar. Der Kunststoff kann entweder opak oder transparent sein. Das Rohrverteilerstück 68 ist so ausgelegt, dass es den erforderlichen Prüfdrücken (z. B. in der Größenordnung von 10 bar) sicher standhält.

Im Vergleich zu herkömmlichen Schlauchverbindungen und auch bekannten einfachen Verbindungsstücken ist das Rohrverteilerstück 68, das gemäß dem in Figur 11 gezeigten Ausführungsbeispiel drei Abzweigungen 70 aufweist, mit stark abknickendem Strömungsverlauf ausgebildet. Genauer gesagt weist das Rohrverteilerstück 68 hier an seinen beiden Längsenden jeweils eine Abbiegung 72 in Form eines 90°-Knicks auf. Die Abbiegungen 72 ergeben sich also nicht durch Zusammensetzen einzelner Teile, sondern sind bereits Bestandteil des Rohrverteilerstücks 68.

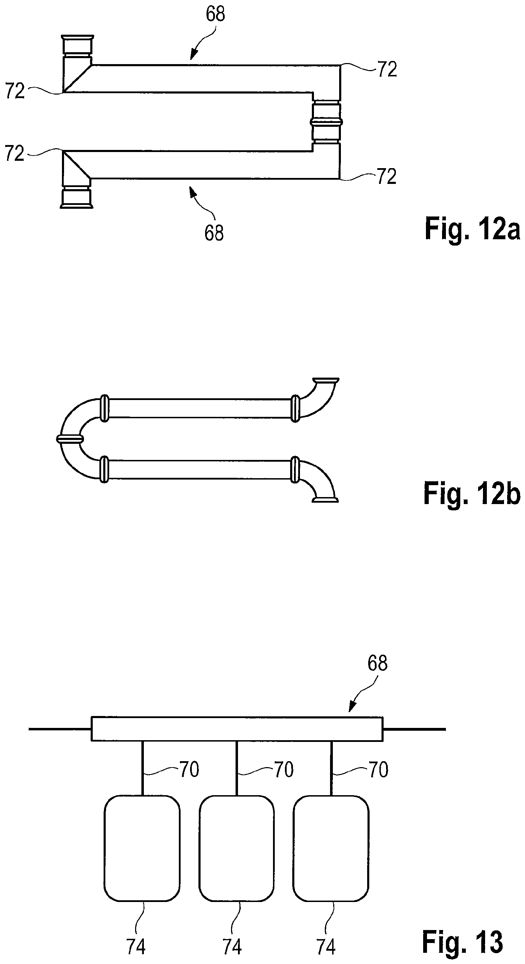

Die Figuren 12a und 12b zeigen einen Vergleich zwischen einer Kombination zweier Rohrverteilerstücke 68 (Figur 12a) und einer Kombination herkömmlicher Rohrverteilerstücke (Figur 12b), wie sie jeweils für den Anschluss von sechs Filtercapsulen 12 an den Abzweigungen 70 benötigt werden. Der Vergleich zeigt, dass im Gegensatz zu der Kombination herkömmlicher Rohrverteilerstücke nur zwei Rohrverteilerstücke 68 miteinander verbunden werden müssen und eine signifikante Bauraumeinsparung erreicht wird.

Die Verbindung zweier Rohrverteilerstücke 68 kann beispielsweise mittels TRI-Clamp-Verbindungen erfolgen. An die Enden des Rohrverteilerstücks 68 bzw. dessen Abzweigungen 70 können auch direkt Sterilkonnektoren angebracht werden. Ferner sind auch andere Verbindungsarten möglich, wie z. B. Verschrauben oder Verschweißen. Die Verbindungen sind in allen Fällen so druckstabil, dass sie den erforderlichen Prüfdrücken standhalten. Da weniger Verbindungsstellen als bei Kombinationen nach Stand der Technik vorhanden sind, ist die Gefahr, dass die Rohrverteilerstücke 68 sich gegeneinander verdrehen, nicht gegeben. Somit ist die mechanische Belastung der Filtercapsulen 12 minimiert.

Selbstverständlich sind auch andere Ausführungsformen des Rohrverteilerstücks 68 mit nur einer oder mehreren, ggf. auch anders verlaufenden integrierten Abbiegungen 72 und/oder Abzweigungen 70 möglich, die die gleichen und ggf. noch weitere Vorteile im Gesamtzusammenhang aufweisen.

Grundsätzlich lassen sich Ventile oder andere Komponenten direkt an die offenen Enden bzw. die Abzweigungen 70 der Rohrverteilerstücke 68 anschließen, sodass der Kern einer Einweg-Filtrationsvorrichtung 10 ohne Schläuche aufgebaut werden kann und somit deutlich druckstabiler ist.

Figur 13 zeigt beispielhaft eine mögliche andere Anwendung des Rohrverteilerstücks 68 in Verbindung mit Einweg-Behältern 74, z. B. in Form von Beuteln.

Wie bereits erwähnt, lassen sich die verschiedenen Aspekte der Erfindung, soweit sinnvoll, beliebig miteinander kombinieren.

Der Einsatz der verschiedenen Ausführungsformen der Einweg-Filtrationsvorrichtung 10 und deren Komponenten, insbesondere des Rohrverteilerstücks 68, ist nicht auf den (bio-)pharmazeutischen Bereich beschränkt.

### Bezugszeichenliste

- 10: Einweg-Filtrationsvorrichtung
- 12: Filtercapsule
- 12a: Sterilfiltercapsule
- 12b: Vorfiltercapsule
- 12c-12e: andere Filtercapsulen
- 14: Halterung
- 16: Seitenwand
- 18: Querstrebe
- 20: Haltemittel
- 22: Rohrleitung
- 24: Abzweigung
- 26: Standfuß
- 28: Filterkerze
- 30: Zulaufeinrichtung
- 32: Ablaufeinrichtung
- 34: Außenanschluss
- 36: Verbindungsbauteil
- 38: Verschluss
- 40: Eingang
- 42: Ausgang
- 44: erstes Abzweigungsglied
- 46: zweites Abzweigungsglied
- 48: erstes Absperrventil
- 50: Luftfilter für Integritätstest
- 52: zweites Absperrventil
- 54: Abfallbehälter
- 56: drittes Absperrventil
- 58: Einweg-Kontrolleinheit
- 60: viertes Absperrventil
- 62: Luftfilter zum Entlüften
- 64: Schutzfilter
- 66: Wagen
- 68: Rohrverteilerstück
- 70: Abzweigung
- 72: Abbiegung
- 74: Einweg-Behälter

## Patentansprüche

1. Einweg-Filtrationsvorrichtung (10), mit einer Mehrzahl durch starre Leitungen miteinander verbundener Einweg-Filtercapsulen (12), von denen wenigstens ein Teil in einem durch eine Halterung (14) universell vorgegebenen Raster fest angebracht sind, wobei die Filtercapsulen (12), insbesondere hinsichtlich Filterart, Bauart und/oder Baugröße, und/oder die Verbindungen der Filtercapsulen (12) für einen gewünschten Filtrationsprozess vorkonfiguriert sind, wobei die Leitungen mehrere Leitungszweige mit zugehörigen Filtercapsulen (12) bilden, die nacheinander oder parallel durchströmt werden, **dadurch gekennzeichnet, dass** an einem oberen Außenanschluss (34) der Einweg-Filtrationsvorrichtung (10) ein sterilisierbarer Luftfilter (62) zum Entlüften der Einweg-Filtrationsvorrichtung (10) angeordnet ist, wobei ein Teil des Strömungswegs zwischen dem Außenanschluss (34) und dem Luftfilter (62) als Schauglas ausgebildet ist, oder in dem Strömungsweg zwischen dem Außenanschluss (34) und dem Luftfilter (62) wenigstens ein Indikator angeordnet ist, der auf Wasser reagiert, oder ein Teil des Strömungswegs zwischen dem Außenanschluss (34) und dem Luftfilter (62) durch einen wenigstens teilweise durchsichtigen Silikonschlauch gebildet ist, der von einer durchsichtigen stützenden Hülle umgeben ist.

2. Einweg-Filtrationsvorrichtung (10) nach Anspruch 1, **dadurch gekennzeichnet, dass** sich wenigstens einige der Filtercapsulen (12) hinsichtlich Filterart, Bauart und/oder Baugröße unterscheiden.

3. Einweg-Filtrationsvorrichtung (10) nach Anspruch 1 oder 2, **dadurch gekennzeichnet, dass** das Raster ein 3x3-Raster für maximal neun Filtercapsulen (12) ist und die Leitungen so vorkonfiguriert sind, dass drei oder weniger in einer Reihe des Rasters angeordnete Filtercapsulen (12) einem Leitungszweig zugehörig sind.

4. Einweg-Filtrationsvorrichtung (10) nach einem der vorhergehenden Ansprüche, **dadurch gekennzeichnet, dass** wenigstens zwei parallele Leitungszweige einen gemeinsamen Eingang (40) und/oder einen gemeinsamen Ausgang (42) haben.

5. Einweg-Filtrationsvorrichtung (10) nach einem der vorhergehenden Ansprüche, **dadurch gekennzeichnet, dass** alle Filtercapsulen (12) Sterilfiltercapsulen (12b) sind.

6. Einweg-Filtrationsvorrichtung (10) nach einem der Ansprüche 1 bis 4, **dadurch gekennzeichnet, dass** alle Filtercapsulen (12) Vorfiltercapsulen (12a) sind.

7. Einweg-Filtrationsvorrichtung (10) nach einem der Ansprüche 1 bis 4, **dadurch gekennzeichnet, dass** eine Kombination von wenigstens einer Sterilfiltercapsule (12a) und wenigstens einer Vorfiltercapsulen (12a) vorgesehen ist.

8. Einweg-Filtrationsvorrichtung (10) nach einem der vorhergehenden Ansprüche, **dadurch gekennzeichnet, dass** in einem nachgeschalteten Leitungszweig eine Kontroll-Filtereinrichtung, vorzugsweise in Form einer Sterilfiltercapsule (12b), angeordnet ist.

9. Einweg-Filtrationsvorrichtung (10) nach einem der Ansprüche 1 bis 7, **dadurch gekennzeichnet, dass** in mehreren nachgeschalteten parallelen Leitungszweigen jeweils eine Kontroll-Filtereinrichtung, vorzugsweise in Form einer Sterilfiltercapsule (12b), angeordnet ist.

10. Einweg-Filtrationsvorrichtung (10) nach Anspruch 8 oder 9, **dadurch gekennzeichnet, dass** die Kontroll-Filtereinrichtung Teil einer Baugruppe ist, die für einen separaten Integritätstest der Kontroll-Filtereinrichtung vorgesehen ist und einen sterilisierbaren Luftfilter (50) umfasst.

11. Einweg-Filtrationsvorrichtung (10) nach Anspruch 10, **dadurch gekennzeichnet, dass** an einen Zulauf und an einen Ablauf der Kontroll-Filtereinrichtung ein zulaufseitiges erstes Abzweigungsglied (44) bzw. ein ablaufseitiges zweites Abzweigungsglied (46) angeschlossen ist, wobei an eines der beiden freien Enden des ersten Abzweigungsglieds (44) über ein zwischengeschaltetes erstes Absperrventil (48) der Ausgang des sterilisierbaren Luftfilters (50) angeschlossen ist, wobei an eines der beiden freien Enden des zweiten Abzweigungsglieds (46) über ein zwischengeschaltetes zweites Absperrventil (52) optional ein Abfallbehälter (54) angeschlossen ist, wobei mit dem anderen freien Ende des ersten Abzweigungsglieds (44) über ein zwischengeschaltetes drittes Absperrventil (56) die Baugruppe aus der Kontroll-Filtereinrichtung, dem Luftfilter (50), dem optionalen Abfallbehälter (54), dem ersten und dem zweiten Absperrventil (48, 52) sowie den beiden Abzweigungsgliedern (44, 46) mit einem Außenanschluss (34) der Einweg-Filtrationsvorrichtung (10) verbunden ist.

12. Einweg-Filtrationsvorrichtung (10) nach Anspruch 11, **dadurch gekennzeichnet, dass** das andere freie Ende des zweiten Abzweigungsglieds (46) einen Filtratausgang der Einweg-Filtrationsvorrichtung (10) bildet, der durch ein viertes Absperrventil (60) verschließbar ist.

13. Einweg-Filtrationsvorrichtung (10) nach einem der Ansprüche 8 bis 12, **dadurch gekennzeichnet, dass** die Kontroll-Filtereinrichtung in einem äußeren Leitungszweig angeordnet ist.

14. Einweg-Filtrationsvorrichtung (10) nach einem der Ansprüche 8 bis 12, **dadurch gekennzeichnet, dass** die Kontroll-Filtereinrichtung außerhalb des vorgegebenen Rasters für die Filtercapsulen (12) angeordnet ist.

## Claims

1. Single-use filtration device (10), comprising a plurality of single-use filter capsules (12) which are connected to each other by rigid lines and at least some of which are fixedly mounted in a grid that is universally predefined by a holder (14), wherein the filter capsules (12), in particular with regard to the type of filter, the type of construction and/or the size, and/or the connections of the filter capsules (12) are preconfigured for a desired filtration process, wherein the lines form a plurality of line branches with associated filter capsules (12), through which a flow passes successively or in parallel, **characterized in that** a sterilizable air filter (62) for venting the single-use filtration device (10) is arranged at an upper external port (34) of the single-use filtration device (10), wherein a portion of the flow path between the external port (34) and the air filter (62) is designed as a sight-glass, or at least one indicator which reacts to water is arranged in the flow path between the external port (34) and the air filter (62), or a portion of the flow path between the external port (34) and the air filter (62) is formed by an at least partially transparent silicone tube which is surrounded by a transparent supporting casing.

2. Single-use filtration device (10) according to claim 1, **characterized in that** at least some of the filter capsules (12) differ with regard to the type of filter, the type of construction and/or the size.

3. Single-use filtration device (10) according to claim 1 or 2, **characterized in that** the grid is a 3×3 grid for a maximum of nine filter capsules (12), and the lines are preconfigured such that three or less filter capsules (12) arranged in a row of the grid belong to a line branch.

4. Single-use filtration device (10) according to any one of the preceding claims, **characterized in that** at least two parallel line branches have a common inlet (40) and/or a common outlet (42).

5. Single-use filtration device (10) according to any one of the preceding claims, **characterized in that** all the filter capsules (12) are sterile filter capsules (12b).

6. Single-use filtration device (10) according to any one of claims 1 to 4, **characterized in that** all the filter capsules (12) are prefilter capsules (12a).

7. Single-use filtration device (10) according to any one of claims 1 to 4, **characterized in that** a combination of at least one sterile filter capsule (12a) and at least one prefilter capsule (12a) is provided.

8. Single-use filtration device (10) according to any one of the preceding claims, **characterized in that** a control filter means, preferably in the form of a sterile filter capsule (12b), is arranged in a downstream line branch.

9. Single-use filtration device (10) according to any one of claims 1 to 7, **characterized in that** a control filter means, preferably in the form of a sterile filter capsule (12b), is arranged in each case in a plurality of downstream parallel line branches.

10. Single-use filtration device (10) according to claim 8 or 9, **characterized in that** the control filter means is part of an assembly which is provided for a separate integrity test of the control filter means and which includes a sterilizable air filter (50).

11. Single-use filtration device (10) according to claim 10, **characterized in that** an inflow-side first branching member (44) and an outflow-side second branching member (46) are connected to an inflow and to an outflow of the control filter means, respectively, wherein the outlet of the sterilizable air filter (50) is connected to one of the two free ends of the first branching member (44) via an interposed first shut-off valve (48), wherein optionally a waste container (54) is connected to one of the two free ends of the second branching member (46) via an interposed second shut-off valve (52), and wherein the assembly comprising the control filter means, the air filter (50), the optional waste container (54), the first and the second shut-off valve (48, 52) and the two branching members (44, 46) is connected to an external port (34) of the single-use filtration device (10) by the other free end of the first branching member (44) via an interposed third shut-off valve (56).

12. Single-use filtration device (10) according to claim 11, **characterized in that** the other free end of the second branching member (46) forms a filtrate outlet of the single-use filtration device (10), which can be closed by a fourth shut-off valve (60).

13. Single-use filtration device (10) according to any one of claims 8 to 12, **characterized in that** the control filter means is arranged in an outer line branch.

14. Single-use filtration device (10) according to any one of claims 8 to 12, **characterized in that** the control filter means is arranged outside of the predefined grid for the filter capsules (12).

## Revendications

1. Dispositif de filtration à usage unique (10), avec une pluralité de capsules de filtre à usage unique (12) reliées ensemble par des conduites rigides dont au moins une partie sont montées de façon fixe dans un cadre prédéfini universellement par un support (14), dans lequel les capsules de filtre (12), en particulier en matière de type de filtre, de type de construction et/ou de taille de construction, et/ou les liaisons des capsules de filtre (12) sont préconfigurés pour un processus de filtration souhaité, dans lequel les conduites forment plusieurs branches de conduite avec des capsules de filtre (12) correspondantes qui sont traversées par écoulement les unes après les autres ou parallèlement, **caractérisé en ce qu'**un filtre à air (62) stérilisable est agencé contre un raccord extérieur (34) supérieur du dispositif de filtration à usage unique (10) pour l'aération du dispositif de filtration à usage unique (10), dans lequel une partie du trajet d'écoulement entre le raccord extérieur (34) et le filtre à air (62) est conçu en tant que voyant, ou au moins un indicateur, qui réagit à l'eau, est agencé dans le trajet d'écoulement entre le raccord extérieur (34) et le filtre à air (62), ou une partie du trajet d'écoulement entre le raccord extérieur (34) et le filtre à air (62) est formé par un tuyau en silicone au moins partiellement transparent qui est entouré par une gaine de soutien transparente.

2. Dispositif de filtration à usage unique (10) selon la revendication 1, **caractérisé en ce qu'**au moins certaines des capsules de filtre (12) se différencient quant au type de filtre, au type de construction et/ou à la taille de construction.

3. Dispositif de filtration à usage unique (10) selon la revendication 1 ou 2, **caractérisé en ce que** le cadre est un cadre 3x3 pour neuf capsules de filtre (12) au maximum et les conduites sont configurées de sorte que trois ou moins des capsules de filtre (12) agencées dans une rangée du cadre appartiennent à une branche de conduite.

4. Dispositif de filtration à usage unique (10) selon l'une des revendications précédentes, **caractérisé en ce qu'**au moins deux branches de conduite parallèles présentent une entrée (40) commune et/ou une sortie (42) commune.

5. Dispositif de filtration à usage unique (10) selon l'une des revendications précédentes, **caractérisé en ce que** toutes les capsules de filtre (12) sont des capsules de filtre stérile (12b).

6. Dispositif de filtration à usage unique (10) selon l'une des revendications 1 à 4, **caractérisé en ce que** toutes les capsules de filtre (12) sont des capsules de préfiltre (12a).

7. Dispositif de filtration à usage unique (10) selon l'une des revendications 1 à 4, **caractérisé en ce qu'**est prévue une combinaison d'au moins une capsule de filtre stérile (12a) et d'au moins une capsule de préfiltre (12a).

8. Dispositif de filtration à usage unique (10) selon l'une des revendications précédentes, **caractérisé en ce qu'**un équipement de filtre de contrôle, de préférence sous la forme d'une capsule de filtre stérile (12b), est agencé dans une branche de conduite montée en aval.

9. Dispositif de filtration à usage unique (10) selon l'une des revendications 1 à 7, **caractérisé en ce qu'**un équipement de filtre de contrôle, de préférence sous la forme d'une capsule de filtre stérile (12b), est respectivement agencé dans plusieurs branches de conduite parallèles montées en aval.

10. Dispositif de filtration à usage unique (10) selon la revendication 8 ou 9, **caractérisé en ce que** l'équipement de filtre de contrôle fait partie d'un groupe de construction qui est prévu pour un test d'intégrité séparé de l'équipement de filtre de contrôle et comprend un filtre à air (50) stérilisable.

11. Dispositif de filtration à usage unique (10) selon la revendication 10, **caractérisé en ce qu'**à une alimentation et à une évacuation de l'équipement de filtre de contrôle est raccordé respectivement un premier organe d'embranchement (44) côté alimentation et un second organe d'embranchement (46) côté évacuation, dans lequel à l'une des deux extrémités libres du premier organe d'embranchement (44) est raccordée la sortie du filtre à air (50) stérilisable via une première vanne d'arrêt (48) montée de façon intermédiaire, dans lequel à l'une des deux extrémités libres du second organe d'embranchement (46) est optionnellement raccordé un récipient à déchets (54) via une deuxième vanne d'arrêt (52) montée de façon intermédiaire, dans lequel avec l'autre extrémité libre du premier organe d'embranchement (44) le groupe de construction constitué de l'équipement de filtre de contrôle, du filtre à air (50), du récipient à déchets (54) optionnel, de la première et de la deuxième vanne d'arrêt (48, 52) ainsi que des deux organes d'embranchement (44, 46) est relié à un raccord extérieur (34) du dispositif de filtration à usage unique (10) via une troisième vanne d'arrêt (56) montée de façon intermédiaire.

12. Dispositif de filtration à usage unique (10) selon la revendication 11, **caractérisé en ce que** l'autre extrémité libre du second organe d'embranchement (46) forme une sortie de filtrat du dispositif de filtration à usage unique (10), laquelle sortie de filtrat peut être fermée par une quatrième vanne d'arrêt (60).

13. Dispositif de filtration à usage unique (10) selon l'une des revendications 8 à 12, **caractérisé en ce que** l'équipement de filtre de contrôle est agencé dans une branche de conduite extérieure.

14. Dispositif de filtration à usage unique (10) selon l'une des revendications 8 à 12, **caractérisé en ce que** l'équipement de filtre de contrôle est agencé à l'extérieur du cadre prédéfini pour les capsules de filtre (12).
